(19) **Europäisches Patentamt / European Patent Office / Office européen des brevets**

(11) **EP 4 599 818 A1**

(12) **EUROPEAN PATENT APPLICATION**
published in accordance with Art. 153(4) EPC

(43) Date of publication:
**13.08.2025 Bulletin 2025/33**

(21) Application number: **23874126.8**

(22) Date of filing: **05.10.2023**

(51) International Patent Classification (IPC):
**A61K 8/19** (2006.01)    **A61Q 17/04** (2006.01)
**C01G 33/00** (2006.01)

(52) Cooperative Patent Classification (CPC):
**A61K 8/19; A61Q 17/04; C01G 33/00**

(86) International application number:
**PCT/BR2023/050344**

(87) International publication number:
**WO 2024/073831 (11.04.2024 Gazette 2024/15)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.10.2022 BR 102022020129**

(71) Applicant: **Instituto Hercílio Randon
95181899 Farroupilha (BR)**

(72) Inventors:
• **BOARETTO, Joel**
  **95032-180 Caxias do Sul (BR)**
• **CRUZ, Robinson Carlos Dudley**
  **95020-472 Caxias do Sul (BR)**

(74) Representative: **Currado, Luisa et al
De Simone & Partners S.r.l.
Via Giulio Caccini, 1
00198 Roma (IT)**

(54) **PREMIX AND PROCESS FOR PRODUCING A COSMETIC COMPOSITION, COSMETIC COMPOSITION, METHOD FOR PHOTOPROTECTION OF A SURFACE AND USE OF THE PREMIX**

(57) The present invention is in the fields of Cosmetology and related Sciences. More specifically, the invention relates to a premix for producing a cosmetic composition, a process for producing a cosmetic composition and an improved cosmetic composition. It further defines a method for photoprotection of a surface and using said premix to prepare a cosmetic composition for photoprotection against UV radiation of an individual's skin surface. The premix and composition of the invention comprise niobium nanoparticles and provide advantageous and surprising effects, including high photoprotection power.

Fig. 17

**Description**

**Field of the Invention**

The present invention is in the fields of Cosmetology and related Sciences. More specifically, the invention relates to a premix for producing a cosmetic composition, a process for producing a cosmetic composition and an improved cosmetic composition. It further defines a method for photoprotection of a surface and use of said premix to prepare a cosmetic composition for photoprotection against UV radiation of an individual's skin surface. The premix and composition of the invention comprise niobium nanoparticles and provide advantageous and surprising effects, including high photoprotection power.

**Background of the Invention**

Among the physical photoprotectors available on the market, titanium dioxide is the most commonly used, followed by zinc oxide and hydroxyapatite. In 2019, this ingredient represented a market share of 14.5 million Euros for all its applications; it is a dye used in cosmetics, food, medicines, and paints as a whitening agent.

Although it is the most common physical sunscreen, this ingredient has problems such as: whitish appearance on the skin, pasty feel, poor spreadability, incompatibility with some cosmetic carriers. Furthermore, titanium dioxide has been banned from the European community for use in the food industry, as it is a potentially carcinogenic ingredient.

The present invention provides an alternative that solves these problems and also has additional advantages.

Patent document JP2003267850A discloses the use of a powder dispersion (Premix) that includes nanoparticles in a cosmetic composition, more specifically, a sunscreen. On the other hand, said document does not disclose a premix or a composition with the specific characteristics of the present invention.

Patent document WO2021121647A1 discloses the use of a complex metal material, applicable in cosmetic compositions, such as sunscreens having an improvement in the UV absorption process. On the other hand, said document only discloses several examples generically, without specific tests, and does not disclose a premix or a composition with the specific characteristics of the present invention.

Patent document WO2007133808A2 discloses the use of nanoparticles in cosmetic compositions. However, said document does not disclose a premix or a composition with the specific characteristics of the present invention.

The article Ali et al. (01/2022) (Use of niobium oxide nanoparticles as nanofillers in PVP/PVA blends to enhance UV-visible absorption, opto-linear, and nonlinear optical properties) discloses improvement of UV absorption properties with the use of niobium pentoxide nanoparticles. On the other hand, said document does not disclose a premix or a composition with the specific characteristics of the present invention.

The article Lyu et al. (08/2022) (Nanoparticles in sunscreen: exploration of the effect and harm of titanium oxide and zinc oxide) presents the use of nanoparticles in a sunscreen. On the other hand, it only discusses the effect of titanium oxides and zinc oxides and does not disclose a premix or a composition with the specific characteristics of the present invention.

From what can be inferred from the literature searched, no documents were found anticipating or suggesting the teachings of the present creation/secret/invention. The invention disclosed herein has, in the eyes of the inventors, novelty and inventive activity compared to the prior art.

**Summary of the Invention**

The invention provides a premix for producing a cosmetic composition, a process for producing a cosmetic composition and an improved cosmetic composition. Wherein said premix and composition of the invention comprise a mass amount of niobium nanoparticles. In one embodiment, said mass amount of niobium nanoparticles comprises a degree of crystallinity of at least 26%. It further defines a method for photoprotection of a surface and use of said premix to prepare a cosmetic composition for photoprotection against UV radiation of an individual's skin surface.

In one embodiment, the premix can be prepared as a stable colloidal suspension and therefore presents the subsequent advantage for those who add it to a cosmetic formulation. This advantage is achieved by reducing mixing times, regardless of the base, as these differ from $TiO_2$ and other available materials that are supplied as a dry powder, as these require an expensive and laborious dispersion step when incorporated into the final cosmetic mixture.

In the context of the present invention, the expression "niobium nanoparticle" encompasses various chemical entities containing niobium, including metallic niobium, niobium oxides, hydrates, hydrides, carbides, or nitrides, niobium iron or niobium alloyed with other metals or transition metals, or combinations thereof. It also includes niobium pentoxide ($Nb_2O_5$), $NbO_2$, and NbO.

In one embodiment, said mass amount of niobium nanoparticles preferably comprises a degree of crystallinity of at least 41%, preferably a degree of crystallinity of at least 61% and even more preferably a degree of crystallinity of at least 80%.

It is worth adding that, in addition to $Nb_2O_5$ the product may contain other Nb polymorphs, such as $NbO_2$ and these same crystalline phases in the amorphous state, in combinations and proportions between them. Example: a premix can be prepared with $Nb_2O_5$ 98% + $NbO_2$ 2%, being partially crystalline and amorphous. In this case, 66% of this composition is crystalline. For another composition, the premix can be prepared with $Nb_2O_5$ 87% + $NbO_2$ 13%. In this case, 95% of this composition is in the amorphous state.

In one embodiment, niobium pentoxide, due to its physicochemical characteristics, is shown to be an excellent alternative to titanium dioxide. In the present invention, the evaluation of UVA and UVB photoprotection carried out with the product LN10 (Lotion containing niobium pentoxide particles in various versions, e.g., comprising particles with 1h-processing, 6h-processing and 12h-processing, as well as particles with micro and sub-micro diameter), showed advantages over products containing titanium dioxide, zinc oxide and nanohydroxyapatite. The product of the invention provided benefits comparable to titanium dioxide in terms of photoprotection, with advantages in relation to cosmetic sensory, among others.

These and other objects of the invention will be immediately valued by those skilled in the art and by companies with interests in the segment and will be described in sufficient detail for their reproduction, in the following description.

## Brief Description of the Figures

The following figures are presented:

Fig. 1 shows the results of the particle size distribution analysis for the sample with a processing time of 1h in counting frequency (%) by diameter (nm).

Fig. 2 shows the results of the particle size distribution analysis for the sample with a processing time of 3h in counting frequency (%) by diameter (nm).

Fig. 3 shows the results of the particle size distribution analysis for the sample with a processing time of 6h in counting frequency (%) by diameter (nm).

Fig. 4 shows the results of the particle size distribution analysis for the sample with a processing time of 12h in counting frequency (%) by diameter (nm).

Fig. 5 shows the results of the particle size distribution analysis for the sample with a processing time of 6h in percentage (%) of number density by size class ($\mu$m).

Fig. 6 shows the results of the particle size distribution analysis for the sample with a processing time of 6h in percentage (%) of volume density by size class ($\mu$m).

Fig. 7 shows the results of the particle size distribution analysis for the sample with a processing time of 12h in percentage (%) of number density by size class ($\mu$m).

Fig. 8 shows the results of the particle size distribution analysis for the sample with a processing time of 12h in percentage (%) of volume density by size class ($\mu$m).

Fig. 9 shows a comparative result between the curves obtained as described in example 1 for samples of niobium pentoxide nanoparticles with a processing time of 6h, by making a comparison between the numerical distribution and the volumetric distribution.

Fig. 10 shows a comparative result between the curves obtained as described in example 1 for samples of niobium pentoxide nanoparticles with a processing time of 12h, by making a comparison between the numerical distribution and the volumetric distribution.

Fig. 11 shows a comparative result between the number density curves (%) obtained as described in example 1 for samples of niobium pentoxide nanoparticles with processing times of 6h and 12h, by making a comparison between the numerical distribution for the processing time of 6h and the numerical distribution for the processing time of 12h.

Fig. 12 shows a comparative result between the volume density curves (%) obtained as described in example 1 for samples of niobium pentoxide nanoparticles with processing times of 6h and 12h, by making a comparison between the volumetric distribution for the processing time of 6h and the numerical distribution for the processing time of 12h.

Fig. 13 shows the comparative result between all samples/products subjected to UVA and UVB tests. The following are shown: A) 5% LN10 6h lotion; B) 10% LN10 6h lotion; C) 5% LN10 12h lotion; D) 10% LN10 12h lotion; E) 5% zinc oxide lotion; F) 5% nanohydroxyapatite lotion; G) 5% Titanium dioxide lotion.

Fig. 14 shows the comparative result between the samples/products applied at 5% and subjected to UVA and UVB tests. The following are shown: A) 5% LN10 6h lotion; B) 5% LN10 12h lotion; C) 5% zinc oxide lotion; D) 5% nanohydroxyapatite lotion; E) 5% Titanium dioxide lotion.

Fig. 15 shows the comparative result between the LN 10 6h and 12h samples/products applied at 5% and 10% and subjected to UVA and UVB tests. The following are shown: A) 5% LN10 6h lotion; B) 5% LN10 12h lotion; C) 10% LN10 6h lotion; D) 10% LN10 12h lotion.

Fig. 16 is a set of two photos, that is, A) before and B) after applying the product 5% LN10 12h lotion, demonstrating the immediate soft focus effect of disguising imperfections.

Fig. 17 shows a comparative graph of all the formulations tested in the present invention.

## Detailed Description of the Invention

The invention provides a premix for producing a cosmetic composition, a process for producing a cosmetic composition and an improved cosmetic composition. Wherein said premix and composition of the invention comprise a mass amount of niobium nanoparticles. In one embodiment, said mass amount of niobium nanoparticles comprises a degree of crystallinity of at least 26%.

One of the objects of the invention is to provide a premix and a cosmetic composition that provides a soft focus effect, also called a blur effect, which is a soft coverage effect that evens out the surface and reflects light in such a way that we do not notice imperfections. Said premix is useful as a general makeup primer.

In one embodiment, the premix can be prepared as a stable colloidal suspension and therefore presents the subsequent advantage for those who add it to a cosmetic formulation. This advantage is achieved by reducing mixing times, regardless of the base, as these differ from $TiO_2$ and other available materials that are supplied as a dry powder, as these require an expensive and laborious dispersion step when incorporated into the final cosmetic mixture.

One of the objects of the invention is a skin photoprotective cosmetic composition.

In the present invention, the compositions contain niobium nanoparticles.

In the context of the present invention, the expression "niobium nanoparticle" encompasses various chemical entities containing niobium, including metallic niobium, niobium oxides, hydrates, hydrides, carbides, or nitrides, niobium iron or niobium alloyed with other metals or transition metals, or combinations thereof. It also includes niobium pentoxide ($Nb_2O_5$), $NbO_2$, and $NbO$.

In the context of the present invention, the expression "degree of crystallinity" should be understood as the extent to which a material has a predominantly monocrystalline or polycrystalline phase, in contrast to predominantly amorphous phases.

In one embodiment, said mass amount of nanoparticles preferably comprises a degree of crystallinity of at least 5%, more preferably at least 10%, more preferably at least 15%, more preferably at least 20%, more preferably at least 26%, more preferably at least 30%, more preferably at least 35%, more preferably at least 40%, more preferably a degree of crystallinity of at least 41%, more preferably at least 45%, more preferably at least 50% and even more preferably a degree of crystallinity of at least 55%. In one embodiment, the degree of crystallinity is at least 60%, more preferably at least 61%, more preferably at least 65%, more preferably at least 70%, more preferably at least 75%, more preferably at least 80%. In a non-limiting embodiment, the degree of crystallinity is 26%, 41%, or 61%.

In one embodiment, said mass amount of niobium nanoparticles preferably comprises a degree of crystallinity of at least 41%, preferably a degree of crystallinity of at least 61% and even more preferably a degree of crystallinity of at least 80%.

It is worth adding that, in addition to $Nb_2O_5$ the product may contain other Nb polymorphs, such as $NbO_2$ and these same crystalline phases in the amorphous state, in combinations and proportions between them. Non-limiting example: a premix can be prepared with $Nb_2O_5$ 98% + $NbO_2$ 2%, being partially crystalline and amorphous. In this case, 66% of this composition is crystalline. For another composition, the premix can be prepared with $Nb_2O_5$ 87% + $NbO_2$ 13%. In this case, 95% of this composition is in the amorphous state.

In one embodiment, compositions containing niobium pentoxide nanoparticles are called LN10. In some embodiments, said compositions contain up to 5% or up to 10% by weight of said nanoparticles. In some embodiments, niobium pentoxide nanoparticles have a particle size distribution profile that is dependent on processing time. In some embodiments, the cases of 1 hour-processing, 6 hours-processing and 12 hour-processing are exemplified. The samples processed for 6h and 12h have, respectively, the following particle size distribution profiles: d10(6h) = 142 to 197 nm, d50(6h) = 208 to 343 nm, d90(6h) = 350 to 599 nm; and d10(12h) = 80 to 129 nm, d50(12h) = 125 to 247 nm, d90(12h) = 230 to 435 nm.

An object of the present invention is a method for photoprotection of a surface comprising at least one step of applying the cosmetic composition, as defined above, on at least one surface. In one embodiment of the method, said photoprotection is against UV radiation.

In one embodiment of the method, the surface is an object surface or the skin surface. In one embodiment, said surface is the surface of an individual's skin.

It is an object of the present invention the use of the premix as described above for producing a cosmetic composition for photoprotection against UV radiation of an individual's skin surface.

In the context of the present invention, the term "individual" is to be understood as a human being or animal. In one embodiment, the individual can be understood as a human being.

The invention is also defined by the following clauses:

A premix for producing a cosmetic composition comprising one or more excipients and a mass amount of niobium nanoparticles, wherein said mass amount of niobium nanoparticles comprises a degree of crystallinity of at least 26%.

The premix as described above, wherein said mass amount of nanoparticles preferably comprises a degree of crystallinity of at least 5%, more preferably at least 10%, more preferably at least 15%, more preferably at least 20%, more preferably at least 26%, more preferably at least 30%, more preferably at least 35%, more preferably at least 40%, more preferably a degree of crystallinity of at least 41%, more preferably at least 45%, more preferably at least 50% and even more preferably a degree of crystallinity of at least 55%. In one embodiment, the degree of crystallinity is at least 60%, more preferably at least 61%, more preferably at least 65%, more preferably at least 70%, more preferably at least 75%, more preferably at least 80%. In a non-limiting embodiment, the degree of crystallinity is 26%, 41%, or 61%.

The premix as described above, in which said mass amount of niobium nanoparticles preferably comprises a degree of crystallinity of at least 41%, preferably a degree of crystallinity of at least 61% and even more preferably a degree of crystallinity of at least 80%.

The premix as described above, comprising up to 80% by weight of niobium nanoparticles. This high concentration of nanoparticles facilitates the process of producing cosmetic compositions by distributing them among other ingredients/excipients.

In one embodiment, the premix is made up of particles dispersed in an oil phase comprising said ingredients/excipients.

The premix as described above, in which the nanoparticles are niobium pentoxide with a particle size distribution between d10 = 142 to 197 nm, d50 = 208 to 343 nm, d90 = 350 to 599 nm; and d10 = 80 to 129 nm, d50 = 125 to 247 nm, d90 = 230 to 435 nm.

Process for producing a cosmetic composition comprising at least one step of mixing a premix as described above into cosmetic excipients.

Cosmetic composition comprising one or more excipients and a mass amount of niobium nanoparticles, wherein said mass amount of niobium nanoparticles comprises a degree of crystallinity of at least 26%.

Cosmetic composition as described above, wherein said mass amount of nanoparticles preferably comprises a degree of crystallinity of at least 5%, more preferably at least 10%, more preferably at least 15%, more preferably at least 20%, more preferably at least 26%, more preferably at least 30%, more preferably at least 35%, more preferably at least 40%, more preferably a degree of crystallinity of at least 41%, more preferably at least 45%, more preferably at least 50% and even more preferably a degree of crystallinity of at least 55%. In one embodiment, the degree of crystallinity is at least 60%, more preferably at least 61%, more preferably at least 65%, more preferably at least 70%, more preferably at least 75%, more preferably at least 80%. In a non-limiting embodiment, the degree of crystallinity is 26%, 41 %, or 61%.

The cosmetic composition as described above, in which said mass amount of niobium nanoparticles preferably comprises a degree of crystallinity of at least 41%, preferably a degree of crystallinity of at least 61% and even more preferably a degree of crystallinity of at least 80%.

The cosmetic composition as described above comprising up to 10% by weight of niobium nanoparticles. In one embodiment, said composition comprises from 5% to 10% by weight of niobium nanoparticles.

In one embodiment, the composition is made up of particles dispersed in an oil phase comprising said ingredients/excipients.

The cosmetic composition as described above, in which said niobium nanoparticles are made up of niobium pentoxide with a particle size distribution between d10 = 142 to 197 nm, d50 = 208 to 343 nm, d90 = 350 to 599 nm; and d10 = 80 to 129 nm, d50 = 125 to 247 nm, d90 = 230 to 435 nm.

The cosmetic composition as described above with high photoprotection power.

The cosmetic composition as described above with high photoprotection power against UV radiation.

Method for photoprotection of a surface comprising at least one step of applying the cosmetic composition, as defined above, on at least one surface.

The method for photoprotection of a surface as described above wherein said photoprotection is against UV radiation.

Use of the premix as described above for producing a cosmetic composition for photoprotection against UV radiation of an individual's skin surface.

**Example 1** - Analysis of the nanoparticles used in the examples

Particle size distribution analysis was performed for the samples of niobium pentoxide nanoparticle with processing times (milling) of 1h, 6h, and 12h (in the case of 6h and 12h, called LN10 6h and 12h, respectively), which were used in the following examples.

The data show an exponential decay of crystallinity (%) and crystallite size (nm), with the crystallite size (nm) appearing to reach an apparent plateau after 6h of milling. The reduction in crystallinity shows a behavior similar to that of the crystallite size, however, it still shows a significant variation throughout the exposure of niobium pentoxide particles to high-energy milling. Regarding crystallinity, about 61% of monoclinic phase can be observed for the sample subjected to 1h of milling, about 41% of monoclinic phase can be observed for the sample subjected to 6h of milling and about 26% of monoclinic phase for the sample subjected to 12h of milling. More details about the process for obtaining nanoparticles can be found in the patent application BR112023003019 of the inventors of the present application.

The samples with more amorphous characteristics (e.g., the 12h sample) showed an even more significant improvement in the cosmetic composition properties enhancement (having a degree of crystallinity of about 26%). On the other hand, significant effects were also observed for the samples that were subjected to 1h and 6h of milling (having a degree of crystallinity of about 41% and 61%, respectively).

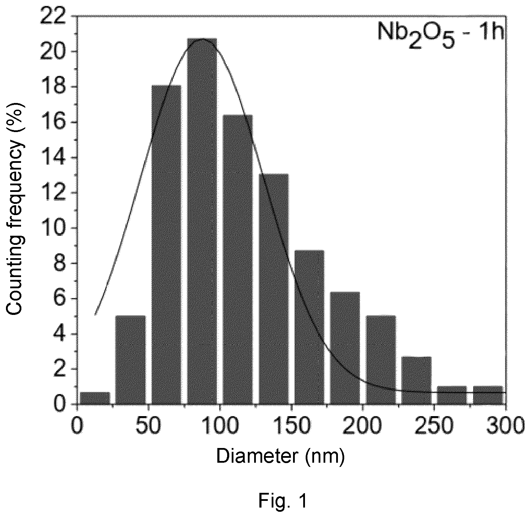

Fig. 1 shows the results of the particle size distribution analysis for the sample with a processing time of 1h in counting frequency (%) by diameter (nm).

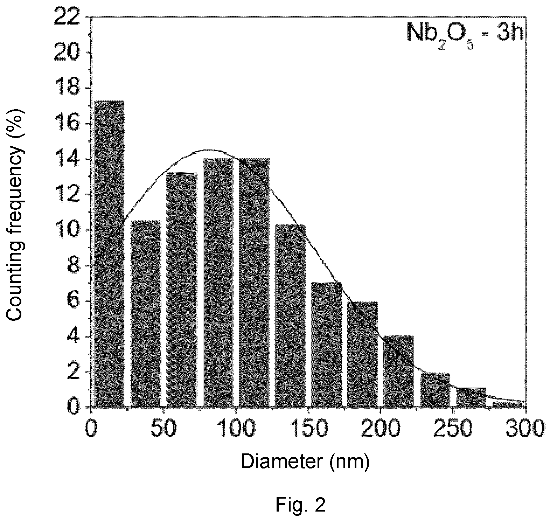

Fig. 2 shows the results of the particle size distribution analysis for the sample with a processing time of 3h in counting frequency (%) by diameter (nm).

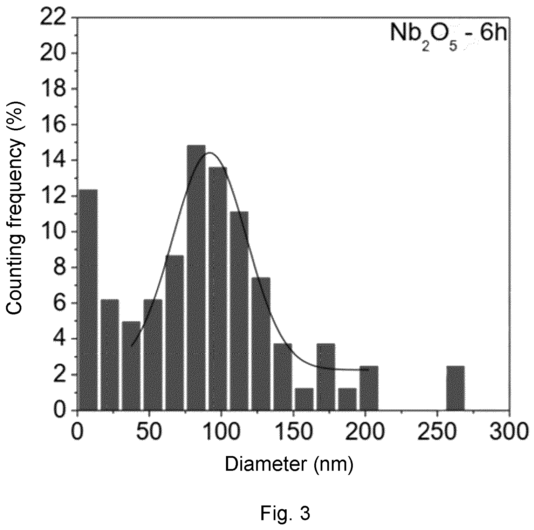

Fig. 3 shows the results of the particle size distribution analysis for the sample with a processing time of 6h in counting frequency (%) by diameter (nm).

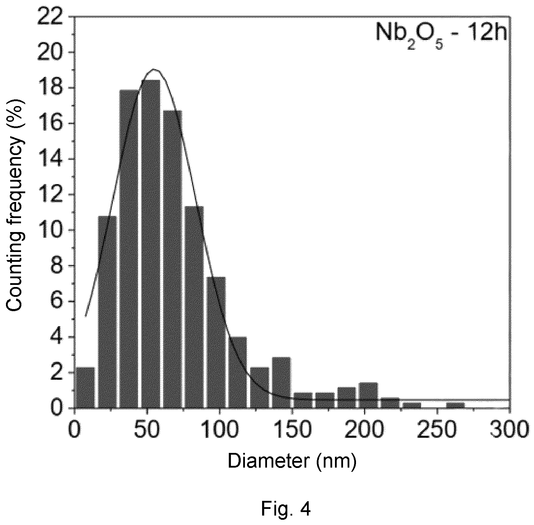

Fig. 4 shows the results of the particle size distribution analysis for the sample with a processing time of 12h in counting frequency (%) by diameter (nm).

Table 1 below shows the parameters of the particle size distribution analysis (numerical distribution) for the sample of niobium pentoxide nanoparticle with a processing time of 6h.

Table 1 - Analysis parameters for the 6h sample.

| | |
|---|---|
| **Particle refractive index** | 2.080 |
| **Particle absorption index** | 0.010 |
| **Dispersant name** | Water |
| **Dispersant refractive index** | 1.330 |
| **Scattering model** | Mie |
| **Analysis model** | General purpose |
| **Weighted residues** | 1.75% |
| **Laser obscuration** | 2.45% |

Table 2 shows the results of the particle size distribution analysis (numerical distribution) for the sample of niobium pentoxide nanoparticle with a processing time of 6h.

Table 2 - Analysis results for the 6h sample.

| | |
|---|---|
| **Concentration** | 0.0001% |
| **Period** | 1.000 |
| **Uniformity** | 0.315 |
| **Specific surface area** | 4140 m$^2$/kg |

(continued)

| D[3;2] | 0.315 $\mu$m |
|---|---|
| D[4;3] | 0.375 $\mu$m |
| Dn(10) | 0.142 $\mu$m |
| Dn(50) | 0.208 $\mu$m |
| Dn(90) | 0.350 $\mu$m |

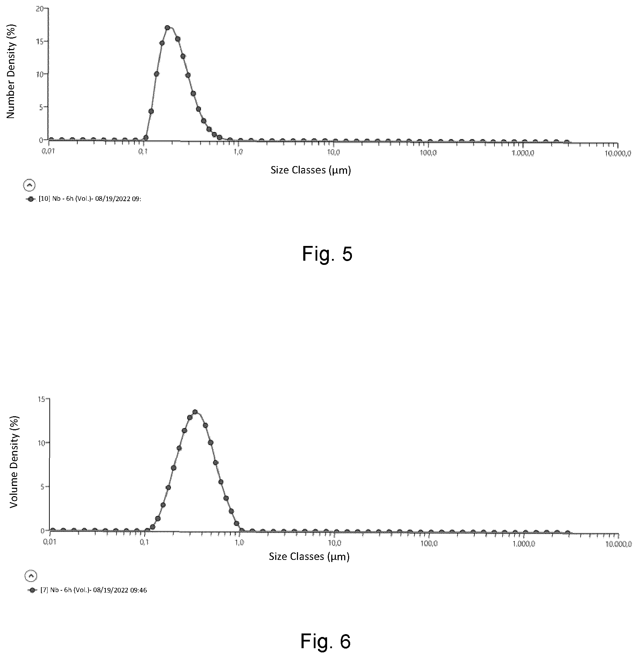

Fig. 5 shows the results of the particle size distribution analysis for the sample with a processing time of 6h in percentage (%) of number density by size class ($\mu$m).
Table 3 details the data serving as the basis for the graph in Fig. 5.

Table 3 - Data from Fig. 5 on analysis for the 6h sample (numerical %)

| Size ($\mu$m) | numerical % |
|---|---|
| 0.010 | 0.00 |
| 0.011 | 0.00 |
| 0.013 | 0.00 |
| 0.015 | 0.00 |
| 0.017 | 0.00 |
| 0.019 | 0.00 |
| 0.022 | 0.00 |
| 0.024 | 0.00 |
| 0.028 | 0.00 |
| 0.032 | 0.00 |
| 0.036 | 0.00 |
| 0.041 | 0.00 |
| 0.046 | 0.00 |
| 0.053 | 0.00 |
| 0.060 | 0.00 |
| 0.068 | 0.00 |
| 0.077 | 0.00 |
| 0.088 | 0.00 |
| 0.100 | 0.01 |
| 0.113 | 3.52 |
| 0.128 | 8.46 |
| 0.146 | 12.47 |
| 0.166 | 14.40 |
| 0.188 | 14.31 |
| 0.214 | 12.87 |
| 0.243 | 10.70 |
| 0.276 | 8.25 |
| 0.314 | 5.91 |
| 0.357 | 3.94 |
| 0.405 | 2.43 |

(continued)

| Size (μm) | numerical % |
|---|---|
| 0.460 | 1.39 |
| 0.523 | 0.73 |
| 0.594 | 0.36 |
| 0.675 | 0.16 |
| 0.767 | 0.07 |
| 0.872 | 0.02 |
| 0.991 | 0.00 |
| 1.125 | 0.00 |
| 1.279 | 0.00 |
| 1.453 | 0.00 |
| 1.651 | 0.00 |
| 1.875 | 0.00 |
| 2.131 | 0.00 |
| 2.421 | 0.00 |
| 2.750 | 0.00 |
| 3.125 | 0.00 |
| 3.550 | 0.00 |
| 4.034 | 0.00 |
| 4.583 | 0.00 |
| 5.207 | 0.00 |
| 5.916 | 0.00 |
| 6.722 | 0.00 |
| 7.637 | 0.00 |
| 8.677 | 0.00 |
| 9.858 | 0.00 |
| 11.201 | 0.00 |
| 12.726 | 0.00 |
| 14.458 | 0.00 |
| 16.427 | 0.00 |
| 18.664 | 0.00 |
| 21.205 | 0.00 |
| 24.092 | 0.00 |
| 27.373 | 0.00 |
| 31.100 | 0.00 |
| 35.335 | 0.00 |
| 40.146 | 0.00 |
| 45.613 | 0.00 |
| 51.823 | 0.00 |
| 58.880 | 0.00 |

(continued)

| Size (μm) | numerical % |
|---|---|
| 66.897 | 0.00 |
| 76.006 | 0.00 |
| 86.355 | 0.00 |
| 98.114 | 0.00 |
| 111.473 | 0.00 |
| 126.652 | 0.00 |
| 143.897 | 0.00 |
| 163.490 | 0.00 |
| 185.752 | 0.00 |
| 211.044 | 0.00 |
| 239.780 | 0.00 |
| 272.430 | 0.00 |
| 309.525 | 0.00 |
| 351.670 | 0.00 |
| 399.555 | 0.00 |
| 453.960 | 0.00 |
| 515.772 | 0.00 |
| 586.001 | 0.00 |
| 665.793 | 0.00 |
| 756.449 | 0.00 |
| 859.450 | 0.00 |
| 976.475 | 0.00 |
| 1109.435 | 0.00 |
| 1260.499 | 0.00 |
| 1432.133 | 0.00 |
| 1627.136 | 0.00 |
| 1848.692 | 0.00 |
| 2100.416 | 0.00 |
| 2386.415 | 0.00 |
| 2711.357 | 0.00 |
| 3080.544 | 0.00 |
| 3500.000 | 0.00 |

Table 4 below shows the parameters of the particle size distribution analysis (volumetric distribution) for the sample of niobium pentoxide nanoparticle with a processing time of 6h.

Table 4 - Analysis parameters for the 6h sample.

| Particle refractive index | 2.080 |
|---|---|
| Particle absorption index | 0.010 |
| Dispersant name | Water |

(continued)

| Dispersant refractive index | 1.330 |
| --- | --- |
| Scattering model | Mie |
| Analysis model | General purpose |
| Weighted residues | 1.75% |
| Laser obscuration | 2.45% |

Table 5 shows the results of the particle size distribution analysis (volumetric distribution) for the sample of niobium pentoxide nanoparticle with a processing time of 6h.

Table 5 - Analysis results for the 6h sample.

| Concentration | 0.0001% |
| --- | --- |
| Period | 1.174 |
| Uniformity | 0.361 |
| Specific surface area | 4140 $m^2$/kg |
| D[3;2] | 0.315 $\mu$m |
| D[4;3] | 0.375 $\mu$m |
| Dv(10) | 0.197 $\mu$m |
| Dv(50) | 0.343 $\mu$m |
| Dv(90) | 0.599 $\mu$m |

Fig. 6 shows the results of the particle size distribution analysis for the sample with a processing time of 6h in percentage (%) of volume density by size class ($\mu$m).
Table 6 details the data serving as the basis for the graph in Fig. 6.

Table 6 - Data from Fig. 6 on analysis for the 6h sample (volumetric %)

| Size ($\mu$m) | volumetric % |
| --- | --- |
| 0.010 | 0.00 |
| 0.011 | 0.00 |
| 0.013 | 0.00 |
| 0.015 | 0.00 |
| 0.017 | 0.00 |
| 0.019 | 0.00 |
| 0.022 | 0.00 |
| 0.024 | 0.00 |
| 0.028 | 0.00 |
| 0.032 | 0.00 |
| 0.036 | 0.00 |
| 0.041 | 0.00 |
| 0.046 | 0.00 |
| 0.053 | 0.00 |
| 0.060 | 0.00 |
| 0.068 | 0.00 |
| 0.077 | 0.00 |

(continued)

| Size (μm) | volumetric % |
|---|---|
| 0.088 | 0.00 |
| 0.100 | 0.00 |
| 0.113 | 0.32 |
| 0.128 | 1.12 |
| 0.146 | 2.41 |
| 0.166 | 4.09 |
| 0.188 | 5.96 |
| 0.214 | 7.86 |
| 0.243 | 9.58 |
| 0.276 | 10.83 |
| 0.314 | 11.39 |
| 0.357 | 11.15 |
| 0.405 | 10.09 |
| 0.460 | 8.43 |
| 0.523 | 6.49 |
| 0.594 | 4.64 |
| 0.675 | 3.09 |
| 0.767 | 1.88 |
| 0.872 | 0.67 |
| 0.991 | 0.00 |
| 1.125 | 0.00 |
| 1.279 | 0.00 |
| 1.453 | 0.00 |
| 1.651 | 0.00 |
| 1.875 | 0.00 |
| 2.131 | 0.00 |
| 2.421 | 0.00 |
| 2.750 | 0.00 |
| 3.125 | 0.00 |
| 3.550 | 0.00 |
| 4.034 | 0.00 |
| 4.583 | 0.00 |
| 5.207 | 0.00 |
| 5.916 | 0.00 |
| 6.722 | 0.00 |
| 7.637 | 0.00 |
| 8.677 | 0.00 |
| 9.858 | 0.00 |
| 11.201 | 0.00 |

(continued)

| Size (μm) | volumetric % |
|-----------|--------------|
| 12.726 | 0.00 |
| 14.458 | 0.00 |
| 16.427 | 0.00 |
| 18.664 | 0.00 |
| 21.205 | 0.00 |
| 24.092 | 0.00 |
| 27.373 | 0.00 |
| 31.100 | 0.00 |
| 35.335 | 0.00 |
| 40.146 | 0.00 |
| 45.613 | 0.00 |
| 51.823 | 0.00 |
| 58.880 | 0.00 |
| 66.897 | 0.00 |
| 76.006 | 0.00 |
| 86.355 | 0.00 |
| 98.114 | 0.00 |
| 111.473 | 0.00 |
| 126.652 | 0.00 |
| 143.897 | 0.00 |
| 163.490 | 0.00 |
| 185.752 | 0.00 |
| 211.044 | 0.00 |
| 239.780 | 0.00 |
| 272.430 | 0.00 |
| 309.525 | 0.00 |
| 351.670 | 0.00 |
| 399.555 | 0.00 |
| 453.960 | 0.00 |
| 515.772 | 0.00 |
| 586.001 | 0.00 |
| 665.793 | 0.00 |
| 756.449 | 0.00 |
| 859.450 | 0.00 |
| 976.475 | 0.00 |
| 1109.435 | 0.00 |
| 1260.499 | 0.00 |
| 1432.133 | 0.00 |
| 1627.136 | 0.00 |

(continued)

| Size (μm) | volumetric % |
|---|---|
| 1848.692 | 0.00 |
| 2100.416 | 0.00 |
| 2386.415 | 0.00 |
| 2711.357 | 0.00 |
| 3080.544 | 0.00 |
| 3500.000 | 0.00 |

Table 7 below shows the parameters of the particle size distribution analysis (numerical distribution) for the sample of niobium pentoxide nanoparticle with a processing time of 12h.

Table 7 - Analysis parameters for the 12h sample.

| Particle refractive index | 2.080 |
|---|---|
| Particle absorption index | 0.010 |
| Dispersant name | Water |
| Dispersant refractive index | 1.330 |
| Scattering model | Mie |
| Analysis model | General purpose |
| Weighted residues | 1.91% |
| Laser obscuration | 2.26% |

Table 8 shows the results of the particle size distribution analysis (numerical distribution) for the sample of niobium pentoxide nanoparticle with a processing time of 12h.

Table 8 - Analysis results for the 12h sample.

| Concentration | 0.0001% |
|---|---|
| Period | 1.200 |
| Uniformity | 0.375 |
| Specific surface area | 6034 $m^2$/kg |
| D[3;2] | 0.216 μm |
| D[4;3] | 0.266 μm |
| Dn(10) | 0.080 μm |
| Dn(50) | 0.125 μm |
| Dn(90) | 0.230 μm |

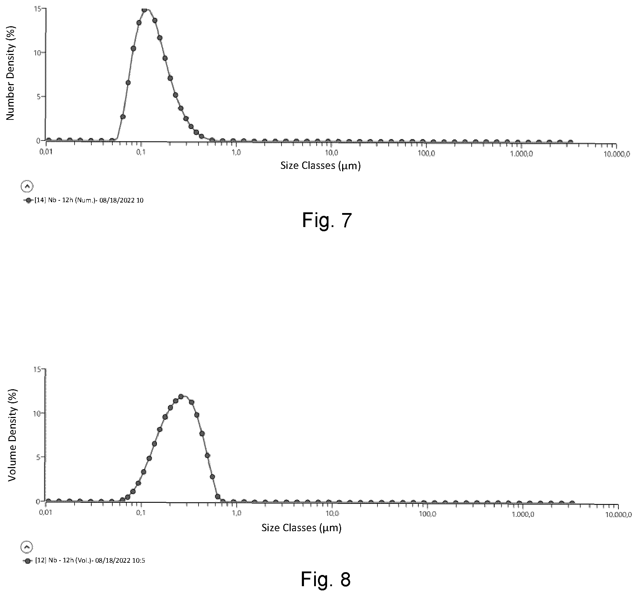

Fig. 7 shows the results of the particle size distribution analysis for the sample with a processing time of 12h in percentage (%) of number density by size class (μm).

Table 9 details the data serving as the basis for the graph in Fig. 7.

Table 9 - Data from Fig. 7 on analysis for the 12h sample (numerical %)

| Size (μm) | numerical % |
|---|---|
| 0.010 | 0.00 |
| 0.011 | 0.00 |
| 0.013 | 0.00 |

(continued)

| Size (μm) | numerical % |
|---|---|
| 0.015 | 0.00 |
| 0.017 | 0.00 |
| 0.019 | 0.00 |
| 0.022 | 0.00 |
| 0.024 | 0.00 |
| 0.028 | 0.00 |
| 0.032 | 0.00 |
| 0.036 | 0.00 |
| 0.041 | 0.00 |
| 0.046 | 0.00 |
| 0.053 | 0.02 |
| 0.060 | 2.17 |
| 0.068 | 5.48 |
| 0.077 | 8.80 |
| 0.088 | 11.26 |
| 0.100 | 12.48 |
| 0.113 | 12.46 |
| 0.128 | 11.43 |
| 0.146 | 9.75 |
| 0.166 | 7.80 |
| 0.188 | 5.92 |
| 0.214 | 4.33 |
| 0.243 | 3.08 |
| 0.276 | 2.11 |
| 0.314 | 1.36 |
| 0.357 | 0.81 |
| 0.405 | 0.43 |
| 0.460 | 0.20 |
| 0.523 | 0.07 |
| 0.594 | 0.01 |
| 0.675 | 0.00 |
| 0.767 | 0.00 |
| 0.872 | 0.00 |
| 0.991 | 0.00 |
| 1.125 | 0.00 |
| 1.279 | 0.00 |
| 1.453 | 0.00 |
| 1.651 | 0.00 |
| 1.875 | 0.00 |

(continued)

| Size (μm) | numerical % |
|---|---|
| 2.131 | 0.00 |
| 2.421 | 0.00 |
| 2.750 | 0.00 |
| 3.125 | 0.00 |
| 3.550 | 0.00 |
| 4.034 | 0.00 |
| 4.583 | 0.00 |
| 5.207 | 0.00 |
| 5.916 | 0.00 |
| 6.722 | 0.00 |
| 7.637 | 0.00 |
| 8.677 | 0.00 |
| 9.858 | 0.00 |
| 11.201 | 0.00 |
| 12.726 | 0.00 |
| 14.458 | 0.00 |
| 16.427 | 0.00 |
| 18.664 | 0.00 |
| 21.205 | 0.00 |
| 24.092 | 0.00 |
| 27.373 | 0.00 |
| 31.100 | 0.00 |
| 35.335 | 0.00 |
| 40.146 | 0.00 |
| 45.613 | 0.00 |
| 51.823 | 0.00 |
| 58.880 | 0.00 |
| 66.897 | 0.00 |
| 76.006 | 0.00 |
| 86.355 | 0.00 |
| 98.114 | 0.00 |
| 111.473 | 0.00 |
| 126.652 | 0.00 |
| 143.897 | 0.00 |
| 163.490 | 0.00 |
| 185.752 | 0.00 |
| 211.044 | 0.00 |
| 239.780 | 0.00 |
| 272.430 | 0.00 |

(continued)

| Size (μm) | numerical % |
|---|---|
| 309.525 | 0.00 |
| 351.670 | 0.00 |
| 399.555 | 0.00 |
| 453.960 | 0.00 |
| 515.772 | 0.00 |
| 586.001 | 0.00 |
| 665.793 | 0.00 |
| 756.449 | 0.00 |
| 859.450 | 0.00 |
| 976.475 | 0.00 |
| 1109.435 | 0.00 |
| 1260.499 | 0.00 |
| 1432.133 | 0.00 |
| 1627.136 | 0.00 |
| 1848.692 | 0.00 |
| 2100.416 | 0.00 |
| 2386.415 | 0.00 |
| 2711.357 | 0.00 |
| 3080.544 | 0.00 |
| 3500.000 | 0.00 |

Table 10 below shows the parameters of the particle size distribution analysis (volumetric distribution) for the sample of niobium pentoxide nanoparticle with a processing time of 12h.

Table 10 - Analysis parameters for the 12h sample.

| Particle refractive index | 2.080 |
|---|---|
| Particle absorption index | 0.010 |
| Dispersant name | Water |
| Dispersant refractive index | 1.330 |
| Scattering model | Mie |
| Analysis model | General purpose |
| Weighted residues | 1.91% |
| Laser obscuration | 2.26% |

Table 11 shows the results of the particle size distribution analysis (volumetric distribution) for the sample of niobium pentoxide nanoparticle with a processing time of 12h.

Table 11 - Analysis results for the 12h sample.

| Concentration | 0.0001% |
|---|---|
| Period | 1.238 |
| Uniformity | 0.382 |

(continued)

| Specific surface area | 6034 m$^2$/kg |
|---|---|
| D[3;2] | 0.216 $\mu$m |
| D[4;3] | 0.266 $\mu$m |
| Dv(10) | 0.129 $\mu$m |
| Dv(50) | 0.247 $\mu$m |
| Dv(90) | 0.435 $\mu$m |

Fig. 8 shows the results of the particle size distribution analysis for the sample with a processing time of 12h in percentage (%) of volume density by size class ($\mu$m).

Table 12 details the data serving as the basis for the graph in Fig. 8.

Table 12 - Data from Fig. 8 on analysis for the 12h sample (volumetric %)

| Size ($\mu$m) | volumetric % |
|---|---|
| 0.010 | 0.00 |
| 0.011 | 0.00 |
| 0.013 | 0.00 |
| 0.015 | 0.00 |
| 0.017 | 0.00 |
| 0.019 | 0.00 |
| 0.022 | 0.00 |
| 0.024 | 0.00 |
| 0.028 | 0.00 |
| 0.032 | 0.00 |
| 0.036 | 0.00 |
| 0.041 | 0.00 |
| 0.046 | 0.00 |
| 0.053 | 0.00 |
| 0.060 | 0.10 |
| 0.068 | 0.39 |
| 0.077 | 0.91 |
| 0.088 | 1.70 |
| 0.100 | 2.77 |
| 0.113 | 4.05 |
| 0.128 | 5.46 |
| 0.146 | 6.83 |
| 0.166 | 8.01 |
| 0.188 | 8.92 |
| 0.214 | 9.57 |
| 0.243 | 9.98 |
| 0.276 | 10.02 |
| 0.314 | 9.48 |
| 0.357 | 8.26 |

(continued)

| Size ($\mu$m) | volumetric % |
|---|---|
| 0.405 | 6.47 |
| 0.460 | 4.37 |
| 0.523 | 2.36 |
| 0.594 | 0.36 |
| 0.675 | 0.00 |
| 0.767 | 0.00 |
| 0.872 | 0.00 |
| 0.991 | 0.00 |
| 1.125 | 0.00 |
| 1.279 | 0.00 |
| 1.453 | 0.00 |
| 1.651 | 0.00 |
| 1.875 | 0.00 |
| 2.131 | 0.00 |
| 2.421 | 0.00 |
| 2.750 | 0.00 |
| 3.125 | 0.00 |
| 3.550 | 0.00 |
| 4.034 | 0.00 |
| 4.583 | 0.00 |
| 5.207 | 0.00 |
| 5.916 | 0.00 |
| 6.722 | 0.00 |
| 7.637 | 0.00 |
| 8.677 | 0.00 |
| 9.858 | 0.00 |
| 11.201 | 0.00 |
| 12.726 | 0.00 |
| 14.458 | 0.00 |
| 16.427 | 0.00 |
| 18.664 | 0.00 |
| 21.205 | 0.00 |
| 24.092 | 0.00 |
| 27.373 | 0.00 |
| 31.100 | 0.00 |
| 35.335 | 0.00 |
| 40.146 | 0.00 |
| 45.613 | 0.00 |
| 51.823 | 0.00 |

(continued)

| Size ($\mu$m) | volumetric % |
|---|---|
| 58.880 | 0.00 |
| 66.897 | 0.00 |
| 76.006 | 0.00 |
| 86.355 | 0.00 |
| 98.114 | 0.00 |
| 111.473 | 0.00 |
| 126.652 | 0.00 |
| 143.897 | 0.00 |
| 163.490 | 0.00 |
| 185.752 | 0.00 |
| 211.044 | 0.00 |
| 239.780 | 0.00 |
| 272.430 | 0.00 |
| 309.525 | 0.00 |
| 351.670 | 0.00 |
| 399.555 | 0.00 |
| 453.960 | 0.00 |
| 515.772 | 0.00 |
| 586.001 | 0.00 |
| 665.793 | 0.00 |
| 756.449 | 0.00 |
| 859.450 | 0.00 |
| 976.475 | 0.00 |
| 1109.435 | 0.00 |
| 1260.499 | 0.00 |
| 1432.133 | 0.00 |
| 1627.136 | 0.00 |
| 1848.692 | 0.00 |
| 2100.416 | 0.00 |
| 2386.415 | 0.00 |
| 2711.357 | 0.00 |
| 3080.544 | 0.00 |
| 3500.000 | 0.00 |

**Example 2** - **Comparison of particle size distribution analyses**

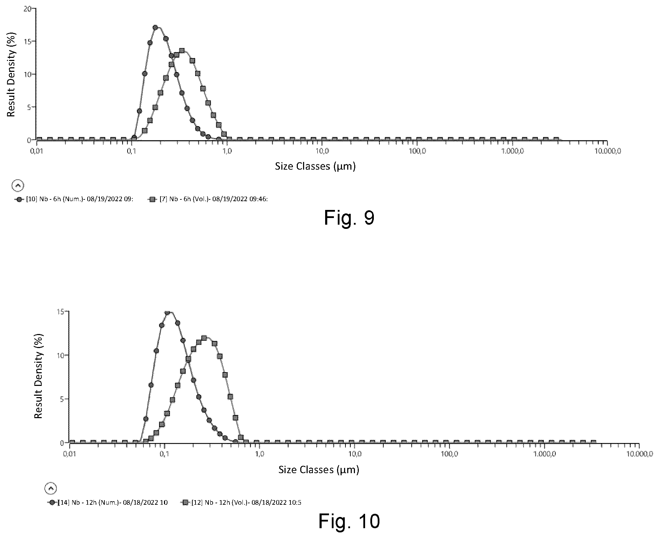

Fig. 9 shows a comparative result between the curves obtained as described in example 1 for samples of niobium pentoxide nanoparticles with a processing time of 6h, by making a comparison between the numerical distribution and the volumetric distribution.

Fig. 10 shows a comparative result between the curves obtained as described in example 1 for samples of niobium pentoxide nanoparticles with a processing time of 12h, by making a comparison between the numerical distribution and the volumetric distribution.

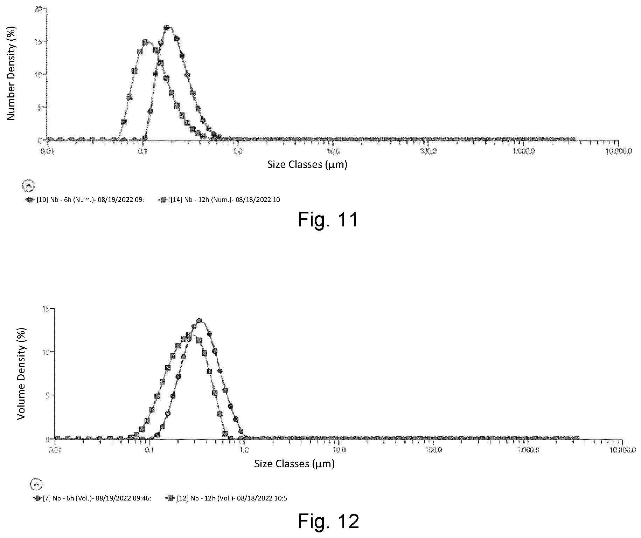

Fig. 11 shows a comparative result between the number density curves (%) obtained as described in example 1 for samples of niobium pentoxide nanoparticles with processing times of 6h and 12h, by making a comparison between the numerical distribution for the processing time of 6h and the numerical distribution for the processing time of 12h.

Fig. 12 shows a comparative result between the volume density curves (%) obtained as described in example 1 for samples of niobium pentoxide nanoparticles with processing times of 6h and 12h, by making a comparison between the volumetric distribution for the processing time of 6h and the numerical distribution for the processing time of 12h.

**Example 3** - **Assessment of UVA and UVB Protection Factor**

The determination of the UVA Protection Factor (UVPF), using a spectrophotometric method, was initially described by BL Diffey and J. Robson and later modified and adapted by COLIPA to assess the protection of the skin against UVA rays provided by a product. The method described in this example consists of evaluating the UVA protection provided by a product by means of a spectrophotometric method, using a substrate suitable for applying the sample. The test is based on measuring the transmittance of UV radiation by the sample that is applied to an appropriate substrate with controlled roughness. The samples are exposed to determined and controlled levels of UV radiation through the use of an irradiation source specified by the COLIPA method. Due to possible variations in interlaboratory results, the *in vivo* SPF is used for the mathematical adjustment of the radiation transmission spectra through a multiplication coefficient C.

The sunscreen sample is exposed to an irradiation dose which is proportional to the initial UVA Protection Factor (FPUVA0), calculated using the unexposed sample transmittance data. The final UVA Protection Factor (FPUVA) is calculated using the adjusted transmittance data of the sample exposed to UV radiation. The method consists of measuring the UV radiation flux through the product, expressed as transmittance, and comparing it with the initial UV radiation flux of the substrate without product as per the spectrophotometric principle below:

$$T(\lambda) = I / I0,$$

where $\lambda$ is the wavelength

Absorbance at a given wavelength $\lambda$ is related to Transmittance by:

$$A(\lambda) = -\log(T(\lambda)$$

The absorbance values are multiplied by different irradiances and action spectra in order to then seek correlations with biological responses obtained by *in vivo* methods. The following are used to calculate SPF: Irradiation source. Spectral irradiance generated by the ultraviolet source $I(\lambda)$. Spectrum of action related to skin response. Action spectrum related to Erythema that expresses the relationship between the cutaneous/subcutaneous reaction and the light energy $E(\lambda)$ (as defined by the CIE "Commission Internationale de l'Eclairage"). In order to calculate FPUVA: Irradiation source. Spectral irradiance generated by the ultraviolet source $I(\lambda)$. Spectrum of action related to skin response. Action spectrum related to Persistent Pigment Darkening (PPD) that expresses the relationship between the cutaneous/subcutaneous reaction and the light energy in the region of 320-400 nm $P(\lambda)$. $I(\lambda)$, $E(\lambda)$, and $P(\lambda)$ are known, and their values are predefined. The UVA Protection Factor (UVPF) is determined by following these steps:

Step 1: Measurement of *in vitro* absorbance $A0(\lambda)$ of the product spread on the PMMA substrate prior to any UV radiation.

Step 2: Mathematical adjustment of the initial UV spectrum using the "C" coefficient to achieve an *in vitro* SPF equal to the *in vivo* SPF of the test sample. Next, the initial UVA Protection Factor (FPUVA0) is calculated using $A0(\lambda)$ and C.

Step 3: A single UV dose is calculated, being proportional to the FPUVA0. (D = FPUVA0 x 1.2 J.cm$^{-2}$).

Step 4: UV radiation of the sample as per the calculated dose D.

Step 5: Measurement of *in vitro* absorbance of the product after UV exposure. Obtaining the second UV absorption spectrum - $A(\lambda)$ data.

Step 6: Mathematical adjustment of the second spectrum (spectrum after exposure to UV radiation) according to the same "C" coefficient previously determined in Step 2. Then, the *in vitro* UVA Protection Factor (UVFP) is calculated.

In this example, one of the purposes is to evaluate the behavior of a sunscreen regarding its effectiveness in UVA protection using the protocol based on the ISO 24443:2012 Determination of sunscreen UVA photoprotection *in vitro* standard.

In this example, the products tested are as per Tables 13-18 below.

Table 13 - 10% LN10 004 12 H5 lotion

| Ingredient/CAS # | % (by weight) |
|---|---|
| Aqua (7732-18-5) | 59.30 |
| Capric/caprylic triglyceride (73398-61-5/65381-09-1) | 10.00 |
| Niobium pentoxide Premix | 10.00 |
| Undecane (1120-21-4) | 7.00 |
| Tridecane (629-50-5) | 3.00 |
| Propanediol (504-63-2/26264-14-2) | 3.00 |
| Potassium cetyl phosphate (84861-79-0/19035-79-1) | 2.50 |
| Glycerin (56-81-5) | 2.00 |
| Cetyl alcohol (36653-82-4) | 1.25 |
| Xanthan gum (11138-66-2) | 0.70 |
| Hydroxyacetophenone (99-93-4) | 0.50 |
| 1,2-Hexanediol (6920-22-5) | 0.25 |
| Caprylyl glycol (1117-86-8) | 0.25 |
| Disodium EDTA (139-33-3/6381-92-6) | 0.10 |
| Tocopheryl acetate (7695-91-2/58-95-7) | 0.10 |
| Sodium hydroxide (1310-73-2) | 0.05 |

Table 14 - 5% LN10 004 6 H lotion

| Ingredient/CAS # | % (by weight) |
|---|---|
| Aqua (7732-18-5) | 64.80 |
| Capric/caprylic triglyceride (73398-61-5/65381-09-1) | 10.00 |
| Niobium pentoxide Premix | 5.00 |
| Undecane (1120-21-4) | 7.00 |
| Tridecane (629-50-5) | 3.00 |
| Propanediol (504-63-2/26264-14-2) | 3.00 |
| Potassium cetyl phosphate (84861-79-0/19035-79-1) | 2.50 |
| Glycerin (56-81-5) | 2.00 |
| Cetyl alcohol (36653-82-4) | 1.25 |
| Xanthan gum (11138-66-2) | 0.70 |
| Hydroxyacetophenone (99-93-4) | 0.50 |
| 1,2-Hexanediol (6920-22-5) | 0.25 |
| Caprylyl glycol (1117-86-8) | 0.25 |
| Disodium EDTA (139-33-3/6381-92-6) | 0.10 |
| Tocopheryl acetate (7695-91-2/58-95-7) | 0.10 |
| Sodium hydroxide (1310-73-2) | 0.05 |

Table 15 - 5% zinc oxide lotion

| Ingredient/CAS # | % (by weight) |
| --- | --- |
| Aqua (7732-18-5) | 60.75 |
| Capric/caprylic triglyceride (73398-61-5/65381-09-1) | 10.00 |
| Undecane (1120-21-4) | 7.00 |
| Zinc oxide/triethoxycapryl solane (1314-13-2/2943-75-1) | 5.00 |
| Tridecane (629-50-5) | 3.00 |
| Propanediol (504-63-2/26264-14-2) | 3.00 |
| Potassium cetyl phosphate (84861-79-0/19035-79-1) | 5.00 |
| Glycerin (56-81-5) | 2.00 |
| Cetyl alcohol (36653-82-4) | 2.50 |
| Xanthan gum (11138-66-2) | 0.70 |
| Hydroxyacetophenone (99-93-4) | 0.50 |
| 1,2-Hexanediol (6920-22-5) | 0.25 |
| Caprylyl glycol (1117-86-8) | 0.25 |
| Disodium EDTA (139-33-3/6381-92-6) | 0.10 |
| Tocopheryl acetate (7695-91-2/58-95-7) | 0.10 |
| Sodium hydroxide (1310-73-2) | 0.05 |

Table 16 - 5% nanohydroxyapatite lotion

| Ingredient/CAS # | % (by weight) |
| --- | --- |
| Aqua (7732-18-5) | 60.75 |
| Capric/caprylic triglyceride (73398-61-5/65381-09-1) | 10.00 |
| Undecane (1120-21-4) | 7.00 |
| Hydroxyapatite (1306-06-05) | 5.00 |
| Tridecane (629-50-5) | 3.00 |
| Propanediol (504-63-2/26264-14-2) | 3.00 |
| Potassium cetyl phosphate (84861-79-0/19035-79-1) | 5.00 |
| Glycerin (56-81-5) | 2.00 |
| Cetyl alcohol (36653-82-4) | 2.50 |
| Xanthan gum (11138-66-2) | 0.50 |
| Hydroxyacetophenone (99-93-4) | 0.50 |
| 1,2-Hexanediol (6920-22-5) | 0.25 |
| Caprylyl glycol (1117-86-8) | 0.25 |
| Disodium EDTA (139-33-3/6381-92-6) | 0.10 |
| Tocopheryl acetate (7695-91-2/58-95-7) | 0.10 |
| Sodium hydroxide (1310-73-2) | 0.05 |

Table 17 - 5% titanium dioxide lotion

| Ingredient/CAS # | % (by weight) |
| --- | --- |
| Aqua (7732-18-5) | 64.50 |
| Capric/caprylic triglyceride (73398-61-5/65381-09-1) | 10.00 |

(continued)

| Ingredient/CAS # | % (by weight) |
|---|---|
| Undecane (1120-21-4) | 7.00 |
| Titanium dioxide (13463-67-7) | 5.00 |
| Tridecane (629-50-5) | 3.00 |
| Propanediol (504-63-2/26264-14-2) | 3.00 |
| Potassium cetyl phosphate (84861-79-0/19035-79-1) | 2.50 |
| Glycerin (56-81-5) | 2.00 |
| Cetyl alcohol (36653-82-4) | 2.50 |
| Xanthan gum (11138-66-2) | 0.50 |
| Hydroxyacetophenone (99-93-4) | 0.50 |
| 1,2-Hexanediol (6920-22-5) | 0.25 |
| Caprylyl glycol (1117-86-8) | 0.25 |
| Disodium EDTA (139-33-3/6381-92-6) | 0.10 |
| Tocopheryl acetate (7695-91-2/58-95-7) | 0.10 |
| Sodium hydroxide (1310-73-2) | 0.05 |

Table 18 - 5% LN10 004 12 H lotion

| Ingredient/CAS # | % (by weight) |
|---|---|
| Aqua (7732-18-5) | 64.80 |
| Capric/caprylic triglyceride (73398-61-5/65381-09-1) | 10.00 |
| Niobium pentoxide premix | 5.00 |
| Undecane (1120-21-4) | 7.00 |
| Tridecane (629-50-5) | 3.00 |
| Propanediol (504-63-2/26264-14-2) | 3.00 |
| Potassium cetyl phosphate (84861-79-0/19035-79-1) | 2.50 |
| Glycerin (56-81-5) | 2.00 |
| Cetyl alcohol (36653-82-4) | 1.25 |
| Xanthan gum (11138-66-2) | 0.20 |
| Hydroxyacetophenone (99-93-4) | 0.50 |
| 1,2-Hexanediol (6920-22-5) | 0.25 |
| Caprylyl glycol (1117-86-8) | 0.25 |
| Disodium EDTA (139-33-3/6381-92-6) | 0.10 |
| Tocopheryl acetate (7695-91-2/58-95-7) | 0.10 |
| Sodium hydroxide (1310-73-2) | 0.05 |

A sample of each product was stored and maintained at IPclin® for 1 month as of the tests completion. Application of the test product to the substrate: the substrate is a material to which the sample is applied. It must be non-fluorescent, photostable, nonreactive and compatible with all ingredients in a formulation. Furthermore, it must distribute the product similarly to what occurs on human skin and, therefore, it must have a textured face. For this method, PMMA (polymethyl methacrylate HD6 - Helioplate) plates with one side of the textured substrate were used. Each product was applied to a substrate in drops evenly distributed along the plate, at a rate of 1.3 mg/cm$^2$. The products were then spread in a standardized manner using a finger protected by a disposable latex finger cot until a uniform film was obtained. The samples were then dried at room temperature for at least 30 minutes in the dark before reading out. A PMMA plate with 5 mg of spread glycerin was used to record the spectrophotometer baseline.

Test procedure. Pre-radiation of plates. A single irradiation dose was calculated from the FPUVA0 value. The samples were kept below 40°C during exposure to UV radiation.

Each PMMA plate was read at different sites to ensure that at least 2 cm² were measured. The PFUVA0 or FPUVA of a plate is calculated through the mean absorbance of all sites on a same PMMA plate. If the coefficient of variation for absorbance between different sites exceeded 50%, this plate would be rejected and a new plate would be prepared. The FPUVA0 or FPUVA of the product is the mean of the FPUVA0 or FPUVA of at least 3 individual plates. If the coefficients of variation between the FPUVA0 or FPUVA of the individual plates exceeded 20%, then new plates would be prepared until the criterion for the coefficient of variation was met. Calculation of critical wavelength: the value of the critical wavelength (Ac) for the test product is defined as that wavelength where the area under the absorbance spectrum for the irradiated product from 290 nm at $\lambda c$ is 90% of the integral of the absorbance spectrum from 290 nm to 400 nm, and is calculated as follows: a series of absorbance values (dependent on the wavelength increment) are calculated for each of the three plates, separately, on which the test product was applied. The results of the products corresponding to Tables 13-18 are shown in Tables 19-24 below.

Table 19a - Results for 10% LN10 004 6 H5 lotion (wherein $\lambda c$ corresponds to the critical wavelength; SD is the standard deviation):

| Plate | FPA$_0$ | Dose (J/cm$^2$) | $\lambda c$ | FPA | Coeff. W | SPF *in vivo* |
|---|---|---|---|---|---|---|
| 1 | 6 | 7.08 | 382 | 6 | 1 | |
| 2 | 5 | 6.55 | 383 | 5 | 1 | |
| 3 | 6 | 6.64 | 383 | 6 | 1 | |
| 4 | 5 | 6.44 | 383 | 5 | 1 | 15 |
| Mean | 5.5 | 6.7 | 382.8 | 5.5 | 1 | |
| SD | 0.6 | 0.3 | 0.5 | 0.6 | 0 | |
| t-Statistics: 3.182446 Number of plates: 4 CI95: 0.918693 % of CI relative to the mean: 16.7 | | | | | | |

The results obtained for the tested standard are shown in Table 19b below (standard: S2, UVA-PF specification between 10.7 and 14.7)

Table 19b - Results for the standard

| Plate | FPA$_0$ | Dose (J/cm$^2$) | FPA | Coeff. W | SPF *in vivo* |
|---|---|---|---|---|---|
| 1 | 11 | 17.22 | 13 | 1.10 | |
| 2 | 13 | 17.48 | 12 | 1.10 | |
| 3 | 13 | 16.54 | 11 | 1.00 | 16 |
| 4 | 14 | 17.1 | 12 | 1.10 | |
| Mean | 12.8 | 17.1 | 12.0 | 1.1 | |
| SD | 1.3 | 0.4 | 0.8 | 0.1 | |
| t-Statistics: 3.182446 Number of plates: 4 CI95: 1.299228 % of CI relative to the mean: 10.8 | | | | | |

The product 10% LN10 004 6 H LOTION, code IPC.2022.3314, showed a mean UVA Protection Factor (FPUVA) of 5.5 and a critical wavelength of 382.8 nm.

Table 20 - Results for 5% LN10 004 06 H lotion (wherein λc corresponds to the critical wavelength; SD is the standard deviation):

| Plate | FPA$_0$ | Dose (J/cm$^2$) | λc | FPA | Coeff. W | SPF *in vivo* |
|---|---|---|---|---|---|---|
| 1 | 5 | 5.29 | 381 | 4 | 1 | |
| 2 | 5 | 5.72 | 381 | 4 | 1 | |
| 3 | 4 | 4.66 | 381 | 5 | 1 | |
| 4 | 4 | 4.97 | 382 | 4 | 1 | 10 |
| Mean | 4.5 | 5.2 | 381.3 | 4.3 | 1 | |
| SD | 0.6 | 0.5 | 0.5 | 0.5 | 0 | |
| t-Statistics: 3.182446 Number of plates: 4 CI95: 0.795612 % of CI relative to the mean: 18.7 | | | | | | |

The results obtained for the tested standard are those shown in Table 19b.

The product 5% LN10 004 6 H lotion, code IPC.2022.3315, showed a mean UVA Protection Factor (FPUVA) of 4.3 and a critical wavelength of 381.3 nm.

Table 21 - Results for 5% zinc oxide lotion (wherein λc corresponds to the critical wavelength; SD is the standard deviation):

| Plate | FPA$_0$ | Dose (J/cm$^2$) | λc | FPA | Coeff. W | SPF *in vivo* |
|---|---|---|---|---|---|---|
| 1 | 5 | 6.28 | 381 | 4 | 1 | |
| 2 | 4 | 5.07 | 382 | 4 | 1 | |
| 3 | 4 | 4.91 | 382 | 4 | 1 | |
| 4 | 4 | 5.18 | 382 | 4 | 1 | 10 |
| Mean | 4.3 | 5.4 | 381.8 | 4 | 1 | |
| SD | 0.5 | 0.6 | 0.5 | 0 | 0 | |
| t-Statistics: 3.182446 Number of plates: 4 CI95: 0 % of CI relative to the mean: 0 | | | | | | |

The results obtained for the tested standard are those shown in Table 19b.

The product 5% zinc oxide lotion, code IPC.2022.3316, showed a mean UVA Protection Factor (FPUVA) of 4.0 and a critical wavelength of 381.3 nm.

Table 22 - Results for 5% nanohydroxyapatite lotion (wherein $\lambda c$ corresponds to the critical wavelength; SD is the standard deviation):

| Plate | FPA$_0$ | Dose (J/cm$^2$) | $\lambda c$ | FPA | Coeff. W | SPF *in vivo* |
|---|---|---|---|---|---|---|
| 1 | 4 | 5.25 | 374 | 3 | 1 | |
| 2 | 3 | 3.94 | 374 | 3 | 1 | |
| 3 | 3 | 4.13 | 374 | 3 | 1 | |
| 4 | 4 | 5.05 | 374 | 4 | 1 | 10 |
| Mean | 3.5 | 4.6 | 374 | 3.3 | 1 | |
| SD | 0.6 | 0.7 | 0 | 0.5 | 0 | |
| t-Statistics: 3.182446<br>Number of plates: 4<br>CI95: 0.795612<br>% of CI relative to the mean: 24.5 | | | | | | |

The results obtained for the tested standard are those shown in Table 19b.

The product 5% nanohydroxyapatite lotion, code IPC.2022.3317, showed a mean UVA Protection Factor (FPUVA) of 3.3 and a critical wavelength of 374 nm.

Table 23 - Results for 5% titanium dioxide lotion (wherein $\lambda c$ corresponds to the critical wavelength; SD is the standard deviation):

| Plate | FPA$_0$ | Dose (J/cm$^2$) | $\lambda c$ | FPA | Coeff. W | SPF *in vivo* |
|---|---|---|---|---|---|---|
| 1 | 5 | 6.08 | 382 | 5 | 1 | |
| 2 | 5 | 6.34 | 383 | 4 | 1 | |
| 3 | 5 | 6.50 | 383 | 4 | 1 | |
| 4 | 5 | 6.28 | 383 | 5 | 1 | 10 |
| Mean | 5 | 6.3 | 382.8 | 4.5 | 1 | |
| SD | 0 | 0.2 | 0.5 | 0.5 | 0 | |
| t-Statistics: 3.182446<br>Number of plates: 4<br>CI95: 0.918693<br>% of CI relative to the mean: 20.4 | | | | | | |

The results obtained for the tested standard are those shown in Table 19b.

The product 5% titanium dioxide lotion, code IPC.2022.3318, showed a mean UVA Protection Factor (FPUVA) of 4.5 and a critical wavelength of 382,8 nm.

Table 24 - Results for 5% LN10 004 12 H LOTION (wherein λc corresponds to the critical wavelength; SD is the standard deviation):

| Plate | FPA$_0$ | Dose (J/cm$^2$) | λc | FPA | Coeff. W | SPF *in vivo* |
|-------|---------|-----------------|-----|-----|----------|---------------|
| 1 | 5 | 6.08 | 382 | 4 | 1 | |
| 2 | 5 | 5.29 | 382 | 4 | 1 | |
| 3 | 5 | 5.61 | 382 | 4 | 1 | 10 |
| 4 | 4 | 5.08 | 382 | 5 | 1 | |
| Mean | 4.8 | 5.5 | 382 | 4.3 | 1 | |
| SD | 0.5 | 0.4 | 0 | 0.5 | 0 | |
| t-Statistics: 3.182446 Number of plates: 4 CI95: 0795612 % of CI relative to the mean: 18.7 | | | | | | |

The results obtained for the tested standard are those shown in Table 19b.

The product 5% LN10 004 12 H LOTION, code IPC.2022.3316, showed a mean UVA Protection Factor (FPUVA) of 4.8 and a critical wavelength of 382 nm.

**Example 4** - **Assessment of the sun protection factor**

The solar spectrum is made up of a series of radiations, and almost all of them can act beneficially. However, when the amount of energy absorbed is higher than the tolerable dose, risks are inevitable. The main solar radiations are: Infrared rays, responsible for the sensation of heat and dehydration of the skin during sun exposure; UVA (320-400 nm), tan superficially; however, they contribute to premature skin aging, induced by prolonged sun exposure; they pass through most common glass. Depending on the skin thickness, they can reach dermal tissues, which makes them as dangerous as higher energy wavelengths (UVB). The UVA range can be subdivided into low UVA, 320-340 nm, responsible for the vast majority of the physiological effects of UVA on the skin, and high UVA, 340-400 nm, responsible for very small changes in elastic fibers; UVB (290-320 nm) is considered more harmful than UVA radiation. They have little penetration into the skin, but due to their high energy, they are the most responsible for immediate damage from solar radiation and for much of the delayed damage. They are also responsible for transforming epidermal ergosterol into vitamin D. When in excess, they cause erythema (sunburn), premature aging, and skin cancer, mainly affecting persons with fair skin; UVC (100-290 nm) is quite harmful, does not stimulate tanning and causes sunburn and cancer. Some studies have detected a slow destruction of the ozone layer caused by CFCs (chlorofluorocarbons), a family of gases used until recently as propellants in aerosols and refrigeration. The consequences of this destruction have not yet been fully assessed, but they will undoubtedly have a huge impact on the planet's life. Tanning is a defense against solar radiation, which stimulates the body to produce melanin, a natural skin pigment, thereby reducing the penetration of UVA and UVB radiation. In the first few days of sun exposure, the melanin production mechanism begins to be activated. During this period, sunscreens with higher protection factors should be used, as they will act similarly to melanin, filtering UVA and UVB rays. The purpose of the sun protection factor, or SPF, is to indicate to the consumer the level of protection provided by the product. The great variability of solar radiation and the different skin types require consumers to know their own skin and define which SPF is most suitable for their case. The higher the SPF value, the higher the level of protection. To choose the SPF that is compatible with a certain skin type, you should not take into account the parts of the body that are most exposed to the sun, such as the arms or face, as these areas are in direct and constant contact with the sun and, therefore, respond differently to its effects. The SPF measurement methods are based on the appearance of erythema on the skin. Hence, it becomes necessary to standardize the way of measuring this erythema. Therefore, a minimal erythema dose (MED) is defined as the amount of energy required to produce the first noticeable erythema reaction with clearly defined borders, observed between 16-24 hours after exposure to ultraviolet radiation. The minimal erythematogenous dose (MED) depends on the individual's skin type, the amount of melanin in the skin, and the length and intensity of the incident wave. Individuals can be classified into six groups, which are also called phototypes, which are estimated based on personal history of burns and tanning, after exposure to the midday sun for 30 to 50 minutes, after a period without exposure. Phototypes have the characteristics described in Table 25.

Table 25 - Skin phototypes according to M. A, Patahak (1983):

| Type | Reaction to sun exposure | Skin color |
|------|--------------------------|------------|
| I | Always burns, never tans. In general, people with milky white skin, light eyes and hair, with freckles | White |
| II | Always burns, minimal tanning. White people, with light hair and eyes | White |
| III | Burns moderately. Gradually tans (light tan). Corresponds to the most common phototype of white people | White |
| IV | Burns a little, always tans (moderate tan). People with light brown skin, oriental dark hair and eyes | Light brown |
| V | Rarely burns, always tans intensely. Brown, indigenous and mixed-race people | Brown |
| VI | They never burn. Deeply pigmented | Dark |

The SPF corresponds to how many MED a person can be exposed to the sun without developing erythema. Therefore, SPF is defined as the ratio of the amount of ultraviolet energy required to produce an erythematic dose on protected skin to the energy required to produce an erythematic dose on unprotected skin.

In this example, the purpose of the tests is to determine the Static Sun Protection Factor (SPF) of the test products using the Protocol based on ISO 24444:2019 - Cosmetics - Sun protection test methods - *In vivo* determination of the sun protection factor (SPF).

The selection of participants followed the criteria: Number of participants included in the study 03 phototypes (Fitzpatrick) I to III Sex F and M Age 21 to 46.

*Inclusion criteria:* Age 18 to 65 years; phototypes I to III; Male and female; Individual typological angle (ITA°) greater than 28° - determined by colorimetry.

*Non-inclusion/exclusion criteria:* Skin marks in the experimental area that interfere with the evaluation of possible skin reactions (pigmentation disorders, vascular malformations, scars, increased hair growth, large amounts of ephelis and nevi, sunburn); active dermatoses (local and disseminated) that may interfere with the results of the study; pregnant or lactating women; history of allergic reactions, irritation or intense feelings of discomfort to topical products: cosmetics and medications; participants with a history of allergy to the material used in the study; history of atopy; history of pathologies aggravated or triggered by ultraviolet radiation; persons with immunodeficiencies; kidney, heart or liver transplant recipients; intense sun exposure or tanning session up to 15 days before the initial evaluation; expectation of intense sun exposure or tanning session during the study period; expectation of bathing in the sea, swimming pool or sauna during the study; participants who practice water sports; use of the following systemic topical medications: immunosuppressants, antihistamines, non-steroidal anti-inflammatory drugs, and corticosteroids up to two weeks before selection; treatment with acidic vitamin A and/or its derivatives, oral or topical, up to 1 month before the start of the study; expected vaccination during the study or up to 3 weeks before the study; participation in another study; any condition not mentioned above that, in the investigator's opinion, may compromise the evaluation of the study; history of non-adherence or unwillingness to adhere to the study protocol; professionals directly involved in the implementation of this protocol and their family members.

*Restrictions imposed on participants:* Do not undergo aesthetic or dermatological treatments during the study.

Medications prohibited during the study: Anti-inflammatory drugs; antihistamines; immunosuppressants; acidic vitamin A and derivatives.

The products according to Tables 13 and 18 of Example 3 were used in the tests.

Consent of the Study Participants. The purpose and methodology of the study were explained to the participants, and they signed a Free and Informed Consent Form.

Procedure to determine static SPF (dry). Multiport 601 equipment with 300 W xenon arc lamp (Solar Light Co Inc); PMA2103 SUV LLG detector; PMA2100 photometer/radiometer. Determination of the Minimal Erythematogenous Dose (MED).

The minimal erythematogenous dose (MED) is considered as the amount of radiant energy necessary to produce the least noticeable and unmistakable redness, assessed 16 to 24 hours after exposure. The MED of each participant was determined by a sequence of exposure to ultraviolet light, with intensity (MED/minute) increasing in geometric progression, with each exposure graduated with a 12% increase in relation to the previous one ($1.12n$). After 16-24 hours of irradiation, a local dermatological examination was performed to check for points with the appearance of erythema.

Determination of static SPF (dry). On the back (infraescapular region) of each participant, rectangles measuring 5.0 x 7.0 cm ($35\ cm^2$), called sites, were marked with a surgical pen. The sites were cleaned with dry cotton. In the demarcated sites, $2\ mg/cm^2$ of the samples under evaluation or of the Standard Product were applied, spreading them evenly with a finger wrapped in a finger cot. After the application, participants waited 15 minutes for it to dry. After drying, the site was irradiated, considering the defined time of the individual MED and the expected SPF of the product. 16-24 hours after irradiation, a local dermatological examination was performed to determine the SPF.

SPF calculation. The SPF value is defined by the ratio between the time required to produce a MED on skin protected with a product containing sunscreen (MEDp) and the time required to produce a MED on unprotected skin (MEDu). Thus, SPF is calculated as per Equation 1.

$$SPF = \frac{MED_p \ (protected \ skin)}{MED_u \ (unprotected \ skin)}$$

The SPF value was calculated for each participant. For the panel of participants, the mean SPF, standard deviation and lower limit of the 95% confidence interval were calculated. Tables 26-31 show the results obtained for the product samples of Tables 13-18, respectively, and the standard product P2 (SPF 13.7 to 18.5).

Table 26 - Results for product samples from Table 13. Number of participants included: 3; Number of participants who completed the study: 3.

| Participants | | | MEDn | Refer. Product (P2) | | Test Product | |
|---|---|---|---|---|---|---|---|
| Refer. | Phototype | ITA° | | MEDp | FPSi | MEDp | FPSi |
| 1 | II | 46 | 1.30 | 19.84 | 15.26 | 20.31 | 15.62 |
| 2 | I | 56 | 0.90 | 14.78 | 16.42 | 13.78 | 15.31 |
| 3 | III | 41 | 1.40 | 22.75 | 16.25 | 21.55 | 15.30 |
| Number of participants | | | | 3 | | | 3 |
| SPF = Σ(SPFi)/n | | | | 16.0 | | | 15.4 |
| Standard Deviation | | | | 0.63 | | | 0.16 |
| CI95 | | | | 1.56 | | | 0.40 |
| % of the CI relative to the mean | | | | 9.73 | | | 2.60 |
| CI relative to mean within the limits? | | | | Yes | | | Yes |

The test product in the table above had a mean static SPF of 15.4. The standard product P2, evaluated concomitantly, showed a mean static SPF of 16.0. These results are in accordance with those provided for in ISO Protocol 24444:2019 - Cosmetics - Sun protection test methods - *In vivo* determination of the sun protection factor (SPF).

The product 10% LN10 004 6 H LOTION, code IPC.2022.3314, showed a mean static SPF of 15.4.

Table 27 - Results for product samples from Table 18. Number of participants included: 3; Number of participants who completed the study: 3.

| Participants | | | MEDn | Refer. Product (P2) | | Test Product | |
|---|---|---|---|---|---|---|---|
| Refer. | Phototype | ITA° | | MEDp | FPSi | MEDp | FPSi |
| 1 | II | 46 | 1.30 | 19.84 | 15.26 | 13.56 | 10.43 |
| 2 | I | 56 | 0.90 | 14.78 | 16.42 | 9.21 | 10.23 |
| 3 | III | 41 | 1.40 | 22.75 | 16.25 | 14.18 | 10.13 |
| Number of participants | | | | 3 | | | 3 |
| SPF = Σ(SPFi)/n | | | | 16.0 | | | 10.3 |
| Standard Deviation | | | | 0.63 | | | 0.15 |
| CI95 | | | | 1.56 | | | 0.38 |
| % of the CI relative to the mean | | | | 9.73 | | | 3.68 |
| CI relative to mean within the limits? | | | | Yes | | | Yes |

The test product in the table above had a mean static SPF of 10.3. The standard product P2, evaluated concomitantly, showed a mean static SPF of 16.0. These results are in accordance with those provided for in ISO Protocol 24444:2019 - Cosmetics - Sun protection test methods - *In vivo* determination of the sun protection factor (SPF).

The product 5% LN10 004 6 H LOTION, code IPC.2022.3315, showed a mean static SPF of 10.3.

**Example 5** - **UVA-B SPF effectiveness compared to market filters**

In this example, a comparative SPF UVA-B effectiveness screening was carried out between the LN10 6h and 12h materials and the inorganic market filters titanium dioxide, zinc oxide and nanohydroxyapatite.

The Screening SPF UVA (320-400 nm) and the Static SPF reflect the level of protection against UVB rays (290-320 nm) offered by a cosmetic product after application to dry skin, with a minimum drying time of 15 minutes. Tests were carried out in order to identify the estimated protection factor for LN10 in the 6h- and 12-hour versions when using concentrations similar to those used in the physical photoprotector market.

UVA rays are characterized by superficial tanning of the skin, contributing to its premature aging, induced by prolonged sun exposure. These rays pass through most common glass. Depending on the skin thickness, they can reach dermal tissues, which makes them as dangerous as higher energy wavelengths (UVB). The UVA range can be subdivided into low UVA, 320-340 nm, responsible for the vast majority of the physiological effects of UVA on the skin, and high UVA, 340-400 nm, responsible for very small changes in elastic fibers. UVB rays (290-320 nm) are considered more harmful than UVA radiation. They have little penetration into the skin, but due to their high energy, they are the most responsible for immediate damage from solar radiation and for much of the delayed damage. They are also responsible for transforming epidermal ergosterol into vitamin D. When in excess, they cause erythema (sunburn), premature aging, and skin cancer, mainly affecting persons with fair skin.

For the study concerned, it was decided to carry out comparative tests with micronized titanium dioxide, zinc oxide and nanohydroxyapatite, both with nanometric particle size, at a concentration of 5% (% solids). These ingredients are the most widely used physical or inorganic photoprotectors on the market. In order to understand whether there is a proportional relationship between use concentration and sun protection factor, LN10 6 h and 12 h were also subjected to tests at 10% application (% solids).

Method: A standard base was produced for the tested formulations, compatible with all applications, as detailed in the clinical test reports. For this base, surfactants and emollients frequently used in photoprotector formulations were listed. The active ingredients LN10 6 h and 12 h are shown in the form of a suspension in an aqueous medium and for both, a solids concentration of 20% was considered for calculating the active ingredient percentage.

The results can be summarized in Table 28.

Table 28 - FPUVA, FPUVB, and SPF results

| Product | FPUVA* | Critical wavelength (nm) | FPUVB** SPF*** Mean static |
|---|---|---|---|
| 5% LN10 6h lotion | 4.3 | 381.3 | 10.3 |
| 10% LN10 6h lotion | 5.5 | 382.8 | 15.4 |
| 5% LN10 12h lotion | 4.8 | 382.0 | 10.2 |
| 10% LN10 12h lotion | 7.0 | 385.0 | 20.8 |
| 5% zinc oxide lotion | 4.0 | 381.8 | 10.2 |
| 5% nanohydroxyapatite lotion | 3.3 | 374.0 | 10.2 |
| 5% titanium dioxide lotion | 4.5 | 382.8 | 13.4 |
| * Ultraviolet A protection factor<br>** Ultraviolet B protection factor<br>*** Sun Protection Factor | | | |

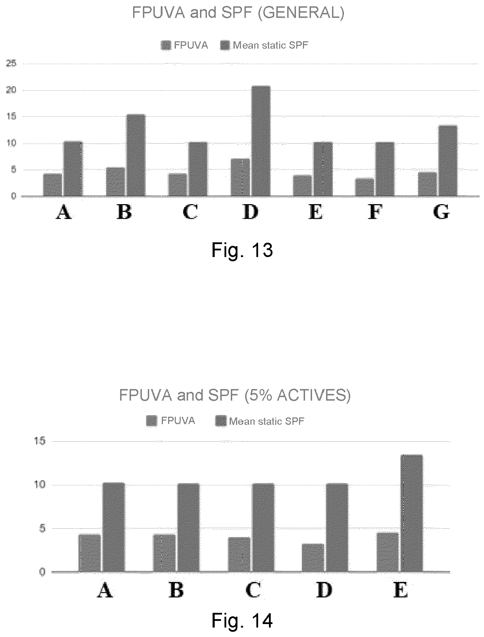

The results are highly promising. Fig. 13 shows an overview of the results in which 5% nanohydroxyapatite showed the lowest absorption for UVA radiation and 10% LN10 showed the highest absorption for UVB (static SPF).

Comparatively, it can be seen in Fig. 14 the results of all active ingredients applied at 5%, in which it can be seen that titanium dioxide shows the best result for UVB absorption (FPUVB = 13.4) and for UVA (FPUVA = 4.5), but for UVA the difference is very small (+ 0.2 FPUVA points) relative to the LN10 6h and 12h samples, both with FPUVA = 4.3 and this result was superior to zinc oxide and nanohydroxyapatite.

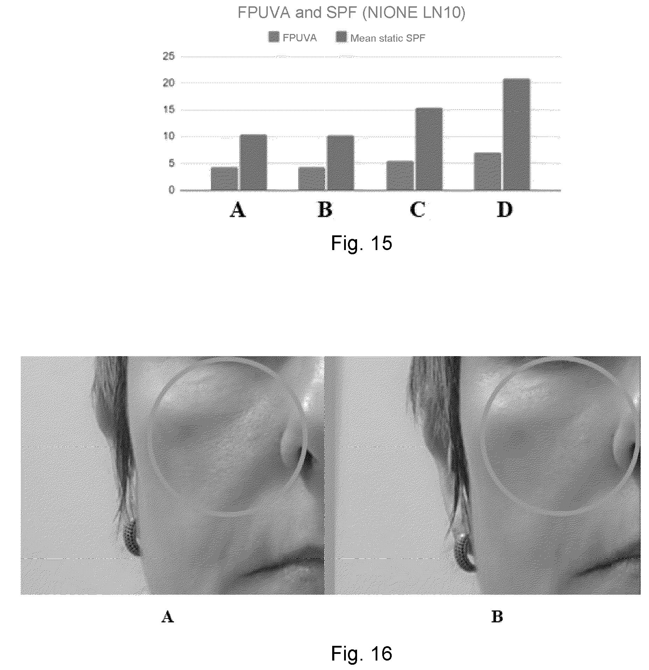

In Fig. 15, it can be seen that among the samples of the product LN10 6h and 12h, the samples applied at 5% showed very similar results, while in the samples applied at 10%, a better performance was observed for LN10 12h. Both LN10 samples showed a FPUVA ratio of at least 1/3 relative to the FPUVB, which complies with the Brazilian legislation, RDC #30 of June 1, 2012 by Anvisa, which describes the Mercosur Technical Regulation on Sunscreens in Cosmetics. From the data obtained for LN10 12h, there is a strong indication that each percentage point is equivalent to 2 FPUVB points.

The results obtained allow us to conclude that LN10 12h at a 10% concentration of active was more promising than LN10 6h at 10% of active. For the comparative concentration of 5%, both LN 10 samples were only less effective in relation to titanium dioxide, but in relation to zinc oxide and nanohydroxyapatite they showed slightly superior performance. These results indicate that LN10 is a very promising material as a physical photoprotector.

**Example 6** - **Comparison with micro-sized niobium particle samples**

For comparison purposes, formulations with micro-sized niobium pentoxide particles (d10 to d100 = 26.2 to 144 $\mu$m) in amounts of 5% and 10% of the composition were also tested.

Table 29 - Sample IPC.2023.1682 - 5% micro Nb particles

| Ingredient/CAS # | % (by weight) |
|---|---|
| Aqua (7732-18-5) | 60.39 |
| Capric/caprylic triglyceride (73398-61-5/65381-09-1) | 10.00 |
| Niobium pentoxide micro premix | 5.00 |
| Coco-caprylate | 5.00 |
| Undecane (1120-21-4) | 3.50 |
| Propanediol (504-63-2/26264-14-2) | 3.00 |
| Cetyl alcohol (36653-82-4) | 3.00 |
| Glycerin (56-81-5) | 2.00 |
| Cetearyl olivate | 1.80 |
| Tridecane (629-50-5) | 1.50 |
| Sorbitan olivate (223706-40-9) | 1.20 |
| Cetearyl alcohol (67762-27-0) | 0.80 |
| Cetyl palmitate (540-10-3) | 0.60 |
| Hydroxyacetophenone (99-93-4) | 0.50 |
| Hydroxyethylcellulose | 0.50 |
| Sorbitan palmitate (5050-91-9) | 0.40 |
| Sorbitan oleate (1338-43-8) | 0.20 |
| 1,2-Hexanediol (6920-22-5) | 0.25 |
| Caprylyl glycol (1117-86-8) | 0.25 |
| Tocopheryl acetate (7695-91-2/58-95-7) | 0.10 |
| Sodium hydroxide (1310-73-2) | 0.01 |

Table 30 - Sample IPC.2023.1683 - 10% micro Nb particles

| Ingredient/CAS # | % (by weight) |
|---|---|
| Aqua (7732-18-5) | 55.39 |
| Capric/caprylic triglyceride (73398-61-5/65381-09-1) | 10.00 |
| Niobium pentoxide micro premix | 10.00 |
| Coco-caprylate | 5.00 |
| Undecane (1120-21-4) | 3.50 |

(continued)

| Ingredient/CAS # | % (by weight) |
|---|---|
| Propanediol (504-63-2/26264-14-2) | 3.00 |
| Cetyl alcohol (36653-82-4) | 3.00 |
| Glycerin (56-81-5) | 2.00 |
| Cetearyl olivate | 1.80 |
| Tridecane (629-50-5) | 1.50 |
| Sorbitan olivate (223706-40-9) | 1.20 |
| Cetearyl alcohol (67762-27-0) | 0.80 |
| Cetyl palmitate (540-10-3) | 0.60 |
| Hydroxyacetophenone (99-93-4) | 0.50 |
| Hydroxyethylcellulose | 0.50 |
| Sorbitan palmitate (5050-91-9) | 0.40 |
| Sorbitan oleate (1338-43-8) | 0.20 |
| 1,2-Hexanediol (6920-22-5) | 0.25 |
| Caprylyl glycol (1117-86-8) | 0.25 |

Using the same methodology as in example 3, the UVA Protection Factor (UVPF) was determined for the samples described in Tables 29 and 30.

Table 31a - Results for sample IPC.2023.1682 - 5% micro Nb particles (wherein $\lambda c$ corresponds to the critical wavelength; SD is the standard deviation):

| Plate | $FPA_0$ | Dose (J/cm$^2$) | $\lambda c$ | FPA | Coeff. W | SPF *in vivo* |
|---|---|---|---|---|---|---|
| 1 | 6 | 7.97 | 377 | 4 | 1 | 10 |
| 2 | 6 | 7.82 | 377 | 4 | 1 | |
| 3 | 5 | 6.67 | 377 | 5 | 1 | |
| 4 | 6 | 7.93 | 377 | 4 | 1 | |
| Mean | 5.75 | 7.60 | 377.00 | 4.25 | 1.00 | |
| SD | 0.50 | 0.62 | 0.00 | 0.50 | 0.00 | |
| t-Statistics: 3.18<br>Number of plates: 4<br>CI95: 0.795612<br>% of CI relative to the mean: 18.72 | | | | | | |

The results obtained for the tested standard are shown in Table 31b below (standard: S2, UVA-PF specification between 10.7 and 14.7)

Table 31b - Results for the standard

| Plate | FPA$_0$ | Dose (J/cm$^2$) | FPA | Coeff. W | SPF *in vivo* |
|---|---|---|---|---|---|
| 1 | 15 | 16.45 | 14 | 1.10 | |
| 2 | 15 | 16.53 | 13 | 1.10 | |
| 3 | 13 | 15.23 | 11 | 1.00 | 16 |
| 4 | 14 | 14.27 | 13 | 1.10 | |
| Mean | 14.3 | 15.6 | 12.8 | 1.1 | |
| SD | 1.0 | 1.1 | 1.3 | 0.1 | |
| t-Statistics: 3.182446<br>Number of plates: 4<br>CI95: 2.002245<br>% of CI relative to the mean: 15.7 | | | | | |

Sample IPC.2023.1682 - 5% micro Nb particles showed a mean UVA protection factor (FPUVA) of 4.25 and a critical wavelength of 377.0 nm.

Table 32a - Results for sample IPC.2023.1683 - 10% micro Nb particles (wherein λc corresponds to the critical wavelength; SD is the standard deviation):

| Plate | FPA$_0$ | Dose (J/cm$^2$) | λc | FPA | Coeff. W | SPF *in vivo* |
|---|---|---|---|---|---|---|
| 1 | 9 | 13.86 | 378 | 8 | 1 | |
| 2 | 9 | 13.73 | 378 | 8 | 1 | |
| 3 | 9 | 14.05 | 378 | 9 | 1 | 20 |
| 4 | 9 | 13.99 | 378 | 9 | 1 | |
| Mean | 9.00 | 13.91 | 378.00 | 8.50 | 1.00 | |
| SD | 0.00 | 0.14 | 0.00 | 0.58 | 0.00 | |
| t-Statistics: 3.18<br>Number of plates: 4<br>C195: 0.918693<br>% of CI relative to the mean: 10.81 | | | | | | |

The results obtained for the tested standard are shown in Table 31b below (standard: S2, UVA-PF specification between 10.7 and 14.7)

Table 31b - Results for the standard

| Plate | FPA$_0$ | Dose (J/cm$^2$) | FPA | Coeff. W | SPF *in vivo* |
|---|---|---|---|---|---|
| 1 | 15 | 16.45 | 14 | 1.10 | |
| 2 | 15 | 16.53 | 13 | 1.10 | |
| 3 | 13 | 15.23 | 11 | 1.00 | 16 |
| 4 | 14 | 14.27 | 13 | 1.10 | |
| Mean | 14.3 | 15.6 | 12.8 | 1.1 | |
| SD | 1.0 | 1.1 | 1.3 | 0.1 | |
| t-Statistics: 3.182446<br>Number of plates: 4<br>CI95: 2.002245<br>% of CI relative to the mean: 15.7 | | | | | |

Sample IPC.2023.1683 - 10% micro Nb particles showed a mean UVA protection factor (FPUVA) of 8.5 and a critical wavelength of 378.0 nm.

Additionally, using the same methodology as in example 4, the sun protection factor of the samples described in Tables 29 and 30 was evaluated.

Table 33 - Results for sample IPC.2023.1682 - 5% micro Nb particles (Table 29). Number of participants included: 10; Number of participants who completed the study: 10.

| Participants | | | Refer. Product (P2) | | Test Product | |
|---|---|---|---|---|---|---|
| Refer. | Phototype | MEDn | MEDp | FPSi | MEDp | FPSi |
| 1 | II | 0.85 | 12.90 | 15.18 | 11.85 | 13.94 |
| 2 | III | 1.35 | 19.38 | 14.36 | 16.12 | 11.94 |
| 3 | III | 1.22 | 17.62 | 14.44 | 15.84 | 12.98 |
| 4 | II | 0.83 | 12.57 | 15.14 | 9.55 | 11.51 |
| 5 | II | 0.82 | 13.06 | 15.93 | 10.38 | 12.66 |
| 6 | III | 1.5 | 21.19 | 14.13 | 18.34 | 12.23 |
| 7 | II | 1.25 | 19.84 | 15.87 | 17.16 | 13.73 |
| 8 | II | 1 | 15.78 | 15.78 | 13.05 | 13.05 |
| 9 | I | 0.9 | 14.75 | 16.39 | 11.13 | 12.37 |
| 10 | III | 1.3 | 19.92 | 15.32 | 16.57 | 12.75 |
| Number of participants | | | 10 | | | 10 |
| SPF = Σ(SPFi)/n | | | 15.3 | | | 12.7 |
| Standard Deviation | | | 0.76 | | | 0.75 |
| CI95 | | | 0.54 | | | 0.54 |
| % of the CI relative to the mean | | | 3.54 | | | 4.25 |
| CI relative to mean within the limits? | | | Yes | | | Yes |

The test sample showed a mean static SPF of 12.7. The standard product P2, evaluated concomitantly, showed a mean static SPF of 15.3. These results are in accordance with those provided for in ISO Protocol 24444:2019 - Cosmetics - Sun protection test methods - *In vivo* determination of the sun protection factor (SPF).

Table 34 - Results for sample IPC.2023.1683 - 10% micro Nb particles (Table 30). Number of participants included: 10; Number of participants who completed the study: 10.

| Participants | | | Refer. Product (P2) | | Test Product | |
|---|---|---|---|---|---|---|
| Refer. | Phototype | MEDn | MEDp | FPSi | MEDp | FPSi |
| 1 | II | 0.85 | 13.90 | 16.35 | 20.25 | 23.82 |
| 2 | III | 1.35 | 20.38 | 15.10 | 31.83 | 23.58 |
| 3 | III | 1.22 | 18.62 | 15.26 | 29.35 | 24.06 |
| 4 | II | 0.83 | 13.57 | 16.35 | 19.66 | 23.69 |
| 5 | II | 0.82 | 14.06 | 17.15 | 20.28 | 24.73 |
| 6 | III | 1.5 | 24.19 | 16.13 | 34.96 | 23.31 |
| 7 | II | 1.25 | 20.84 | 16.67 | 29.17 | 23.34 |
| 8 | II | 1 | 16.78 | 16.78 | 23.52 | 23.52 |
| 9 | I | 0.9 | 14.75 | 16.39 | 22.26 | 24.73 |
| 10 | III | 1.3 | 19.92 | 15.32 | 30.38 | 23.37 |

(continued)

| Participants | | | Refer. Product (P2) | | Test Product | |
|---|---|---|---|---|---|---|
| Refer. | Phototype | MEDn | MEDp | FPSi | MEDp | FPSi |
| Number of participants | | | | 10 | | 10 |
| SPF = Σ(SPFi)/n | | | | 16.2 | | 23.8 |
| Standard Deviation | | | | 0.70 | | 0.53 |
| CI95 | | | | 0.50 | | 0.38 |
| % of the CI relative to the mean | | | | 3.08 | | 1.61 |
| CI relative to mean within the limits? | | | | Yes | | Yes |

The test sample showed a mean static SPF of 23.8. The standard product P2, evaluated concomitantly, showed a mean static SPF of 16.2. These results are in accordance with those provided for in ISO Protocol 24444:2019 - Cosmetics - Sun protection test methods - *In vivo* determination of the sun protection factor (SPF).

**Example 7** - **Comparison with submicro-sized niobium particle samples**

For comparison purposes, formulations with submicro-sized niobium pentoxide particles (d10 to d100 = 0.3 to 5.2 μm) in amounts of 5% and 10% of the composition were also tested.

Table 35 - Sample IPC.2023.1163 - 5% sub-micro Nb particles

| Ingredient/CAS # | % (by weight) |
|---|---|
| Aqua (7732-18-5) | 60.39 |
| Capric/caprylic triglyceride (73398-61-5/65381-09-1) | 10.00 |
| Sub-micro niobium pentoxide premix | 5.00 |
| Coco-caprylate | 5.00 |
| Undecane (1120-21-4) | 3.50 |
| Propanediol (504-63-2/26264-14-2) | 3.00 |
| Cetyl alcohol (36653-82-4) | 3.00 |
| Glycerin (56-81-5) | 2.00 |
| Cetearyl olivate | 1.80 |
| Tridecane (629-50-5) | 1.50 |
| Sorbitan olivate (223706-40-9) | 1.20 |
| Cetearyl alcohol (67762-27-0) | 0.80 |
| Cetyl palmitate (540-10-3) | 0.60 |
| Hydroxyacetophenone (99-93-4) | 0.50 |
| Hydroxyethylcellulose | 0.50 |
| Sorbitan palmitate (5050-91-9) | 0.40 |
| Sorbitan oleate (1338-43-8) | 0.20 |
| 1,2-Hexanediol (6920-22-5) | 0.25 |
| Caprylyl glycol (1117-86-8) | 0.25 |

Table 36 - Sample IPC.2023.1162 - 10% sub-micro Nb particles

| Ingredient/CAS # | % (by weight) |
|---|---|
| Aqua (7732-18-5) | 55.39 |

(continued)

| Ingredient/CAS # | % (by weight) |
|---|---|
| Capric/caprylic triglyceride (73398-61-5/65381-09-1) | 10.00 |
| Sub-micro niobium pentoxide premix | 10.00 |
| Coco-caprylate | 5.00 |
| Undecane (1120-21-4) | 3.50 |
| Propanediol (504-63-2/26264-14-2) | 3.00 |
| Cetyl alcohol (36653-82-4) | 3.00 |
| Glycerin (56-81-5) | 2.00 |
| Cetearyl olivate | 1.80 |
| Tridecane (629-50-5) | 1.50 |
| Sorbitan olivate (223706-40-9) | 1.20 |
| Cetearyl alcohol (67762-27-0) | 0.80 |
| Cetyl palmitate (540-10-3) | 0.60 |
| Hydroxyacetophenone (99-93-4) | 0.50 |
| Hydroxyethylcellulose | 0.50 |
| Sorbitan palmitate (5050-91-9) | 0.40 |
| Sorbitan oleate (1338-43-8) | 0.20 |
| 1,2-Hexanediol (6920-22-5) | 0.25 |
| Caprylyl glycol (1117-86-8) | 0.25 |

Using the same methodology as in example 3, the UVA Protection Factor (UVPF) was determined for the samples described in Tables 35 and 36.

Table 37a - Results for sample IPC.2023.1163 - sub-micro 5% Nb particles (where $\lambda c$ corresponds to the critical wavelength; SD is the standard deviation):

| Plate | $FPA_0$ | Dose (J/cm$^2$) | $\lambda c$ | FPA | Coeff. W | SPF *in vivo* |
|---|---|---|---|---|---|---|
| 1 | 7 | 10.98 | 378 | 6 | 1 | |
| 2 | 7 | 10.93 | 378 | 6 | 1 | |
| 3 | 7 | 11.07 | 378 | 7 | 1 | 10 |
| 4 | 7 | 11.14 | 378 | 7 | 1 | |
| Mean | 7.00 | 11.03 | 378.00 | 6.50 | 1.00 | |
| SD | 0.00 | 0.09 | 0.00 | 0.58 | 0.00 | |

t-Statistics: 3.182446
Number of plates: 4
C195: 2.002245
% of CI relative to the mean: 15.7

The results obtained for the tested standard are shown in Table 37b below (standard: S2, UVA-PF specification between 10.7 and 14.7)

Table 37b - Results for the standard

| Plate | FPA$_0$ | Dose (J/cm$^2$) | FPA | Coeff. W | SPF *in vivo* |
|---|---|---|---|---|---|
| 1 | 15 | 16.45 | 14 | 1.10 | |
| 2 | 15 | 16.53 | 13 | 1.10 | |
| 3 | 13 | 15.23 | 11 | 1.00 | 16 |
| 4 | 14 | 14.27 | 13 | 1.10 | |
| Mean | 14.3 | 15.6 | 12.8 | 1.1 | |
| SD | 1.0 | 1.1 | 1.3 | 0.1 | |
| t-Statistics: 3.182446<br>Number of plates: 4<br>C195: 2.002245<br>% of CI relative to the mean: 15.7 | | | | | |

Sample IPC.2023.1163 - 5% sub-micro Nb particles showed a mean UVA protection factor (FPUVA) of 6.5 and a critical wavelength of 378.0 nm.

Table 38a - Results for sample IPC.2023.1162 - sub-micro 10% Nb particles (where λc corresponds to the critical wavelength; SD is the standard deviation):

| Plate | FPA$_0$ | Dose (J/cm$^2$) | λc | FPA | Coeff. W | SPF *in vivo* |
|---|---|---|---|---|---|---|
| 1 | 10 | 11.29 | 379 | 9 | 1 | |
| 2 | 10 | 10.35 | 379 | 9 | 1 | 20 |
| 3 | 11 | 12.46 | 379 | 9 | 1 | |
| 4 | 10 | 11.78 | 379 | 8 | 1 | |
| Mean | 10.25 | 11.47 | 379.00 | 8.75 | 1.00 | |
| SD | 0.00 | 0.89 | 0.00 | 0.50 | 0.00 | |
| t-Statistics: 3.18<br>Number of plates: 4<br>CI95: 0.795612<br>% of CI relative to the mean: 9.09 | | | | | | |

The results obtained for the tested standard are shown in Table 38b below (standard: S2, UVA-PF specification between 10.7 and 14.7)

Table 38b - Results for the standard

| Plate | FPA$_0$ | Dose (J/cm$^2$) | FPA | Coeff. W | SPF *in vivo* |
|---|---|---|---|---|---|
| 1 | 15 | 16.45 | 14 | 1.10 | |
| 2 | 15 | 16.53 | 13 | 1.10 | |
| 3 | 13 | 15.23 | 11 | 1.00 | 16 |
| 4 | 14 | 14.27 | 13 | 1.10 | |
| Mean | 14.3 | 15.6 | 12.8 | 1.1 | |
| SD | 1.0 | 1.1 | 1.3 | 0.1 | |
| t-Statistics: 3.182446<br>Number of plates: 4<br>C195: 2.002245<br>% of CI relative to the mean: 15.7 | | | | | |

Sample IPC.2023.1162 - 10% sub-micro Nb particles showed a mean UVA protection factor (FPUVA) of 8.75 and a critical wavelength of 379.0 nm.

Additionally, using the same methodology as in example 4, the sun protection factor of the samples described in Tables 35 and 36 was evaluated.

Table 39 - Results for sample IPC.2023.1163 - sub-micro 5% Nb particles (Table 35). Number of participants included: 3; Number of participants who completed the study: 3.

| Participants | | | | Refer. Product (P2) | | Test Product | |
|---|---|---|---|---|---|---|---|
| Refer. | Phototype | ITA° | MEDn | MEDp | FPSi | MEDp | FPSi |
| 1 | I | 62 | 0.60 | 9.68 | 16.13 | 8.32 | 13.87 |
| 2 | II | 45 | 1.25 | 20.53 | 16.42 | 15.56 | 12.45 |
| 3 | III | 36 | 1.60 | 26.72 | 16.70 | 22.37 | 13.98 |
| Number of participants | | | | 3 | | | 3 |
| SPF = Σ(SPFi)/n | | | | 16.4 | | | 13.4 |
| Standard Deviation | | | | 0.29 | | | 0.85 |
| CI95 | | | | 0.71 | | | 2.12 |
| % of the CI relative to the mean | | | | 4.32 | | | 15.82 |
| CI relative to mean within the limits? | | | | YES | | | YES |

The test sample showed a mean static SPF of 13.4. The standard product P2, evaluated concomitantly, showed a mean static SPF of 16.4. These results are in accordance with those provided for in ISO Protocol 24444:2019 - Cosmetics - Sun protection test methods - *In vivo* determination of the sun protection factor (SPF).

Table 40 - Results for sample IPC.2023.1162 - sub-micro 10% Nb particles (Table 36). Number of participants included: 10; Number of participants who completed the study: 10.

| Participants | | | | Refer. Product (P2) | | Test Product | |
|---|---|---|---|---|---|---|---|
| Refer. | Phototype | ITA° | MEDn | MEDp | FPSi | MEDp | FPSi |
| 1 | I | 62 | 0.60 | 9.68 | 16.13 | 14.00 | 23.33 |
| 2 | II | 45 | 1.25 | 20.53 | 16.42 | 30.01 | 24.01 |
| 3 | III | 36 | 1.60 | 26.72 | 16.70 | 37.15 | 23.22 |
| Number of participants | | | | 3 | | | 3 |
| SPF = Σ(SPFi)/n | | | | 16.4 | | | 23.5 |
| Standard Deviation | | | | 0.29 | | | 0.43 |
| CI95 | | | | 0.71 | | | 1.06 |
| % of the CI relative to the mean | | | | 4.32 | | | 4.52 |
| CI relative to mean within the limits? | | | | YES | | | YES |

The test sample showed a mean static SPF of 23.5. The standard product P2, evaluated concomitantly, showed a mean static SPF of 16.4. These results are in accordance with those provided for in ISO Protocol 24444:2019 - Cosmetics - Sun protection test methods - *In vivo* determination of the sun protection factor (SPF).

**Example 8** - **General comparative table**

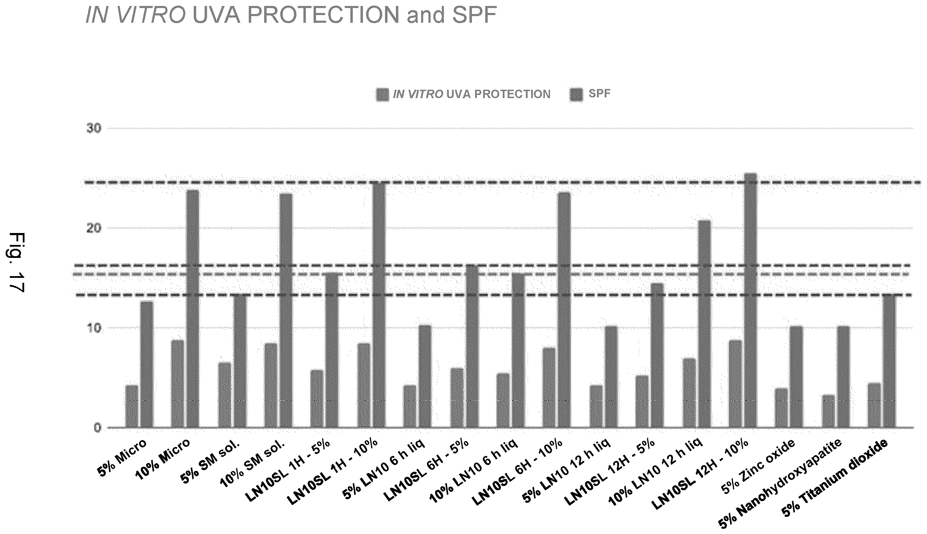

For comparison purposes, Fig. 17 depicts a comparative graph of all the formulations tested in the present invention.

Table 41 - Comparative table for the formulations tested for *in vitro* UVA protection and SPF.

| Sample | Sample characteristics | UVA protection | SPF |
|---|---|---|---|
| 5% Micro | Concentration of 5% by mass of particle dispersed in the formulation | 4.25 | 12.7 |
| 10% Micro | Concentration of 10% by mass of particle dispersed in the formulation | 8.75 | 23.8 |
| 5% sub-micro sol. | Concentration of 5% by mass of particle dispersed in the formulation | 6.50 | 13.4 |
| 10% sub-micro sol. | Concentration of 10% by mass of particle dispersed in the formulation | 8.50 | 23.5 |
| 5% LN10SL 1H | Concentration of 5% solid nanoparticle with 1-hour milling, dispersed in oil phase | 5.75 | 15.5 |
| 10% LN10SL 1H | Concentration of 10% solid nanoparticle with 1-hour milling, dispersed in oil phase | 8.50 | 24.5 |
| 5% LN10 6h liquid | Concentration of 5% nanoparticle in suspension with 6-hour milling, dispersed in aqueous phase. | 4.3 | 10.3 |
| 5% LN10SL 6H | Concentration of 5% solid nanoparticle with 6-hour milling, dispersed in oil phase | 6.00 | 16.3 |
| 10% LN10 6h liquid | Concentration of 10% nanoparticle in suspension with 6-hour milling, dispersed in aqueous phase. | 5.5 | 15.4 |
| 10% LN10SL 6H | Concentration of 10% solid nanoparticle with 6-hour milling, dispersed in oil phase | 8 | 23.6 |
| 5% LN10 12h liquid | Concentration of 5% nanoparticle in suspension with 12-hour milling, dispersed in aqueous phase. | 4.30 | 10.2 |
| 5% LN10SL 12H | Concentration of 5% solid nanoparticle with 12-hour milling, dispersed in oil phase | 5.25 | 14.5 |
| 10% LN10 12h liquid | Concentration of 10% nanoparticle in suspension with 12-hour milling, dispersed in aqueous phase. | 7.00 | 20.8 |
| 10% LN10SL 12H | Concentration of 10% solid nanoparticle with 12-hour milling, dispersed in oil phase | 8.75 | 25.5 |
| 5% Zinc oxide | Concentration of 5% particle by mass dispersed in aqueous phase | 4 | 10.2 |
| 5% Nanohydroxyapatite | Concentration of 5% particle by mass dispersed in aqueous phase | 3.3 | 10.2 |
| 5% Titanium dioxide | Concentration of 5% particle by mass dispersed in aqueous phase | 4.5 | 13.4 |

From the results above, for formulations containing 5% by mass of particles when compared with a composition containing 5% by mass of titanium dioxide (which is commonly used in sunscreens), it is possible to observe that particularly better results than titanium dioxide were observed for compositions containing 5% of niobium pentoxide particles that were subjected to 1h of milling, niobium pentoxide particles that were subjected to 6h of milling and niobium pentoxide particles that were subjected to 12h of milling. Particularly in the case where the solid particles were dispersed in an oil phase.

Likewise, particularly expressive results for higher concentrations (e.g. 10% by mass) were observed for compositions containing niobium pentoxide particles that were subjected to 12h of milling. However, very expressive results at high concentrations were also observed for niobium pentoxide particles that were subjected to 1h of milling, niobium pentoxide particles that were subjected to 6h of milling, as well as particles with micro and sub-micro diameters. For 1h, 6h and 12h particles, particularly in the case where the solid particles were dispersed in an oil phase.

Particularly for the formulation containing particles with micro-diameter, an exfoliating effect was observed.

The applicant, when filing this application with the competent entity, guarantees, seeks and intends to: (i) name the authors/inventors in respect of their respective moral, copyright and patrimonial rights related to their works; (ii) unequivocally indicate that he/she is the holder of the trade or industrial secret and the holder of any form of intellectual property derived therefrom and the applicant wishes; (iii) describe in detail the content of the creations and the secret, proving their existence in the physical and legal environments; (iv) obtain protection for his/her creations of the spirit, as provided for in the Copyright Law; (v) establish the relationship between the examples/embodiments and the creative, ornamental, distinctive or inventive concept according to the applicant's cognition and its context, to clearly demonstrate the scope of his/her protected and/or protectable intangible asset; (vi) request and obtain the additional rights provided for patents, if the applicant chooses to continue with the administrative procedure until the end.

Any future disclosure or publication of this document does not, in itself, constitute authorization for commercial use by third parties. Even if the content becomes part of the *physical* world accessible to third parties, the publication of this document under the terms of the law does not eliminate the *legal* status of secrecy, serving only and solely the spirit of the Law to: (i) serve as proof that the creator created the objects described herein and expressed them in a physical medium, which is this report itself; (ii) unequivocally indicate their owner/holder and authors/inventor(s); (iii) notify third parties of the existence of the creations and said industrial secret, of the content for which intellectual protection is or will be requested under the terms of the Law, including patent protection and the date of its filing, from which priority rights will be granted and the term of validity of the patent exclusivity may begin, if applicable; and (iv) assist in the technological and economic development of the country, from the disclosure of the creation, if this occurs, and the authorization of the use of the secret solely and exceptionally for the purposes of studies and/or development of new improvements, thereby avoiding parallel reinvestment by third parties in the development of the same asset.

It is hereby noted that any commercial use requires authorization from the authors, or the owner/holder and that unauthorized use will result in sanctions provided for by law. In this context, given the extensive detail in which the creation, concept and examples were disclosed by the applicant, those skilled in the art will be able, without much effort, to consider other ways of implementing the present creation and/or invention in ways that are not identical to those merely exemplified above. However, such forms are or may be considered to fall within the scope of one or more of the appended claims.

## Claims

1. Premix for producing a cosmetic composition, **characterized in that** it comprises one or more excipients and a mass amount of niobium nanoparticles, wherein said mass amount of niobium nanoparticles comprises a degree of crystallinity of at least 26%.

2. Premix according to claim 1, **characterized in that** said mass amount of niobium nanoparticles preferably comprises a degree of crystallinity of at least 41%, preferably a degree of crystallinity of at least 61% and even more preferably a degree of crystallinity of at least 80%.

3. Premix according to claim 1, **characterized in that** it comprises up to 80% by weight of niobium nanoparticles.

4. Process for producing a cosmetic composition, **characterized in that** it comprises at least one step of mixing a premix as defined in claim 1 with cosmetic excipients.

5. Cosmetic composition, **characterized in that** it comprises one or more excipients and a mass amount of niobium nanoparticles, wherein said mass amount of niobium nanoparticles comprises a degree of crystallinity of at least 26%.

6. Cosmetic composition according to claim 5, **characterized in that** said mass amount of niobium nanoparticles preferably comprises a degree of crystallinity of at least 41%, preferably a degree of crystallinity of at least 61% and even more preferably a degree of crystallinity of at least 80%.

7. Cosmetic composition according to claim 5, **characterized in that** it comprises up to 10% by weight of niobium nanoparticles.

8. Cosmetic composition according to claim 5, **characterized in that** it has a high photoprotection power.

9. Cosmetic composition according to claim 8, **characterized in that** it has a high photoprotection power against UV radiation.

10. Method for photoprotection of a surface, **characterized in that** it comprises at least one step of applying the cosmetic composition, as defined in claim 5, on at least one surface.

11. Method for photoprotection of a surface according to claim 10, **characterized in that** said photoprotection is against UV radiation.

12. Use of the premix as defined in claim 1, **characterized in that** it is for preparing a cosmetic composition for photoprotection against UV radiation of an individual's skin surface.

Fig. 1

Fig. 2

Fig. 3

Fig. 4

Fig. 5

Fig. 6

Fig. 7

Fig. 8

Fig. 9

Fig. 10

[10] Nb - 6h (Num.)- 08/19/2022 09:        [14] Nb - 12h (Num.)- 08/18/2022 10

Fig. 11

[7] Nb - 6h (Vol.)- 08/19/2022 09:46:        [12] Nb - 12h (Vol.)- 08/18/2022 10:5

Fig. 12

FPUVA and SPF (GENERAL)

Fig. 13

FPUVA and SPF (5% ACTIVES)

Fig. 14

FPUVA and SPF (NIONE LN10)

Fig. 15

Fig. 16

IN VITRO UVA PROTECTION and SPF

Fig. 17

EP 4 599 818 A1

52

## INTERNATIONAL SEARCH REPORT

| International application No. |
|---|
| **PCT/BR2023/050344** |

**A. CLASSIFICATION OF SUBJECT MATTER**

A61K 8/19 (2006.01)i; A61Q 17/04 (2006.01)i; C01G 33/00 (2006.01)
CPC: A61K 8/19; A61Q 17/04; A61K 2800/413; C01G 33/00; C01P 2004/64

According to International Patent Classification (IPC) or to both national classification and IPC

**B. FIELDS SEARCHED**

Minimum documentation searched (classification system followed by classification symbols)

A61K 8/19; A61Q 17/04; C01G 33/00
CPC: A61K 8/19; A61Q 17/04; A61K 2800/413; C01G 33/00; C01P 2004/64

Documentation searched other than minimum documentation to the extent that such documents are included in the fields searched

Base de dados do INPI, PUBMED

Electronic data base consulted during the international search (name of data base and, where practicable, search terms used)

Derwent Innovation, Google Patents, Espacenet, STN (BIOSIS, CAPLUS, KOSMED)

**C. DOCUMENTS CONSIDERED TO BE RELEVANT**

| Category* | Citation of document, with indication, where appropriate, of the relevant passages | Relevant to claim No. |
|---|---|---|
| Y | US 2006039875 A1 (KNOWLAND JOHN S [GB]) 23 February 2006 (2006-02-23)<br>The whole document | 1 - 12 |
| Y | FR 2683145 A1 (OREAL [FR]) 07 May 1993 (1993-05-07)<br>The whole document | 1 - 12 |
| Y | US 2003219391 A1 (OREAL [FR]) 27 November 2003 (2003-11-27)<br>The whole document | 1 - 12 |
| Y | WO 2020031079 A1 (SEKISUI PLASTICS [JP]) 13 February 2020 (2020-02-13)<br>The whole document | 1 - 12 |
| Y | US 9044400 B2 (FELTIN CHARLOTTE [FR]) 02 June 2015 (2015-06-02)<br>The whole document | 1 - 12 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **13 December 2023** | **18 January 2024** |

| Name and mailing address of the ISA/BR | Authorized officer |
|---|---|
| **National Institute of Industrial Property (Brazil)**<br>**Rua Mayrink Veiga, 9, 6º andar, CEP 20.090-910 Rio de Janeiro – RJ**<br>**Brazil** | **Rodrigo BARBOSA FERRARO** |
| Telephone No. **(55 21) 3037-3742, 3037-3984** | Telephone No. **+55 21 3037 4528 - 3037 3319** |

Form PCT/ISA/210 (second sheet) (July 2022)

**EP 4 599 818 A1**

### INTERNATIONAL SEARCH REPORT

| International application No. |
| --- |
| **PCT/BR2023/050344** |

| C. | DOCUMENTS CONSIDERED TO BE RELEVANT | |
| --- | --- | --- |
| Category* | Citation of document, with indication, where appropriate, of the relevant passages | Relevant to claim No. |
| Y | Lenzmann, F. & Shklover, Valery & Brooks, Keith & Graetzel, Michael. (December 2000). Mesoporous Nb2O5 Films: Influence of Degree of Crystallinity on Properties. Journal of Sol-gel Science and Technology - 19. 175-180. Accessed on 13/12/2023. https://doi.org/10.1023/A:1008767717625 - The whole document. | 1 - 12 |

Form PCT/ISA/210 (second sheet) (July 2022)

54

## INTERNATIONAL SEARCH REPORT
### Information on patent family members

International application No.

**PCT/BR2023/050344**

| Patent document cited in search report | | | Publication date (day/month/year) | Patent family member(s) | | | Publication date (day/month/year) |
|---|---|---|---|---|---|---|---|
| US | 2006039875 | A1 | 23 February 2006 | AU | 4273299 | A | 13 December 1999 |
| | | | | AU | 765456 | B2 | 18 September 2003 |
| | | | | DE | 69929799 | D1 | 20 April 2006 |
| | | | | DE | 69929799 | T2 | 31 August 2006 |
| | | | | EP | 1079796 | A1 | 07 March 2001 |
| | | | | EP | 1079796 | B1 | 08 February 2006 |
| | | | | EP | 1598056 | A1 | 23 November 2005 |
| | | | | ES | 2255292 | T3 | 16 June 2006 |
| | | | | GB | 9811377 | D0 | 22 July 1998 |
| | | | | JP | 2002516347 | A | 04 June 2002 |
| | | | | JP | 4162857 | B2 | 08 October 2008 |
| | | | | US | 6869596 | B1 | 22 March 2005 |
| | | | | US | 2005169857 | A1 | 04 August 2005 |
| | | | | US | 8642019 | B2 | 04 February 2014 |
| | | | | WO | 9960994 | A1 | 02 December 1999 |
| FR | 2683145 | A1 | 07 May 1993 | NONE | | | |
| US | 2003219391 | A1 | 27 November 2003 | JP | 2003267850 | A | 25 September 2003 |
| WO | 2020031079 | A1 | 13 February 2020 | NONE | | | |
| US | 9044400 | B2 | 02 June 2015 | US | 2012282188 | A1 | 08 November 2012 |
| | | | | BR | 112012012343 | A2 | 01 March 2017 |
| | | | | BR | 112012012343 | B1 | 08 August 2017 |
| | | | | CN | 102844014 | A | 26 December 2012 |
| | | | | CN | 102844014 | B | 03 February 2016 |
| | | | | EP | 2503983 | A2 | 03 October 2012 |
| | | | | EP | 2503983 | B1 | 23 October 2013 |
| | | | | ES | 2441559 | T3 | 05 February 2014 |
| | | | | FR | 2952815 | A1 | 27 May 2011 |
| | | | | FR | 2952815 | B1 | 06 January 2012 |
| | | | | WO | 2011064720 | A2 | 03 June 2011 |
| | | | | WO | 2011064720 | A3 | 04 August 2011 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003267850 A
- WO 2021121647 A1
- WO 2007133808 A2
- BR 112023003019

**Non-patent literature cited in the description**

- *CHEMICAL ABSTRACTS*, 7732-18-5
- *CHEMICAL ABSTRACTS*, 73398-61-5
- *CHEMICAL ABSTRACTS*, 65381-09-1
- *CHEMICAL ABSTRACTS*, 1120-21-4
- *CHEMICAL ABSTRACTS*, 629-50-5
- *CHEMICAL ABSTRACTS*, 504-63-2
- *CHEMICAL ABSTRACTS*, 26264-14-2
- *CHEMICAL ABSTRACTS*, 84861-79-0
- *CHEMICAL ABSTRACTS*, 19035-79-1
- *CHEMICAL ABSTRACTS*, 56-81-5
- *CHEMICAL ABSTRACTS*, 36653-82-4
- *CHEMICAL ABSTRACTS*, 11138-66-2
- *CHEMICAL ABSTRACTS*, 99-93-4
- *CHEMICAL ABSTRACTS*, 6920-22-5
- *CHEMICAL ABSTRACTS*, 1117-86-8
- *CHEMICAL ABSTRACTS*, 139-33-3
- *CHEMICAL ABSTRACTS*, 6381-92-6
- *CHEMICAL ABSTRACTS*, 7695-91-2
- *CHEMICAL ABSTRACTS*, 58-95-7
- *CHEMICAL ABSTRACTS*, 1310-73-2
- *CHEMICAL ABSTRACTS*, 1314-13-2
- *CHEMICAL ABSTRACTS*, 2943-75-1
- *CHEMICAL ABSTRACTS*, 1306-06-05
- *CHEMICAL ABSTRACTS*, 13463-67-7
- *CHEMICAL ABSTRACTS*, 223706-40-9
- *CHEMICAL ABSTRACTS*, 67762-27-0
- *CHEMICAL ABSTRACTS*, 540-10-3
- *CHEMICAL ABSTRACTS*, 5050-91-9
- *CHEMICAL ABSTRACTS*, 1338-43-8
- *CHEMICAL ABSTRACTS*, 7695-91-2/58-95-7